# EUROPEAN PATENT APPLICATION

(11) **EP 1 795 613 A1**
(43) Date of publication of application: **13.06.2007**
(21) Application number: 05780513.7
(22) Date of filing: 22.08.2005
(51) Int. Cl.: C12Q 1/68, C12N 15/09, C12Q 1/02, C12Q 1/26

(54) **BIOSENSOR WITH THE USE OF PIGMENT-SYNTHESIS GENE OF PURPLE NON-SULFUR BACTERIUM AND METHOD OF CONSTRUCTING THE BIOSENSOR**

(30) Priority: 24.08.2004 JP 2004243163; 10.05.2005 JP 2005136762
(71) Applicant: THE KANSAI ELECTRIC POWER CO., INC., Osaka-shi, Osaka 530-8270 (JP); OSAKA UNIVERSITY, Suita-shi, Osaka 565-0871 (JP)
(72) Inventor: YAGI, Kiyohito; c/o Osaka University,, Osaka 5650871 (JP); KAWASE, Masaya; c/o Osaka University,, Osaka 5650871 (JP); MAEDA, Isamu; c/o Osaka University,, Osaka 5650871 (JP); YAMASHIRO, Hidenori; c/o Osaka University,, Osaka 5650871 (JP); MIYASAKA, H.; c/o The Kansai Electr. Power Co Inc., Osaka-shi, Osaka 5308270 (JP)
(74) Representative: Bates, Philip Ian
(86) International application number: PCT/JP2005/015224
(87) International publication number: WO 2006/022235

(57) **Abstract**

A biosensor where measurement is able to be conducted by simpler operations, cost is less expensive and specific chemical substance is able to be visually detected is provided. In accordance with the present invention, there is provided a biosensor for the detection of a specific chemical substance, **characterized in that** it comprises a recombinant purple non-sulfur bacterium wherein sensor vector is introduced into a pigment variant of a purple non-sulfur bacterium being deficient in gene coding for a spheroidene monooxygenase enzyme, and that the sensor vector is a sensor vector where inductive promoter showing a specific response to a specific chemical substance is connected in an operable manner to the upstream region of gene coding for the spheroidene monooxygenase enzyme.

## Description

### Technical Field of the Invention

The present invention relates to a biosensor using a specific microbe and, more particularly, it relates to a biosensor for a visual detection of harmful substances in environment by a simple means utilizing pigment-synthesizing gene of a purple non-sulfur bacterium and to a method for preparing such a biosensor. The present invention particularly relates to a biosensor for detection of arsenic in environment in low cost and by highly sensitive and simple means utilizing pigment-synthesizing gene of a purple non-sulfur bacterium and also to a method for preparing such a biosensor.

### Background Art

At present, in an aqueous environment such as the ocean, rivers and lakes/marshes, pollution by various harmful substances such as agricultural chemicals, heavy metals and endocrine disrupting chemicals is one of the serious problems and there has been a continued demand for removal thereof, improvement of quality of water, etc. in view of environmental cleanup and improvement in public hygiene. In the removal of various harmful substances as such those existing in environment and in the monitoring of the affection of specific chemical substances on environment, means for detecting them and means for measuring the concentration thereof are important at first. With regard to the means for detection and for grasping the concentration as such, a process where samples are collected in the field and subjected to measurement by instruments in laboratories is common but, in that case, it is not possible to trace the changes in concentration within short time. In addition, it is usual that objects to be measured are present as a mixture and, therefore, means such as chromatography is sometimes necessary for their separation and, in addition, since concentration of the object to be measured is usually much diluted, large amount of sample is sometimes necessary. Furthermore, depending upon the type of the aimed chemical substance, there are problems that its analytical operation is troublesome or that powerful chemicals or hazardous reagent may be necessary and, in measuring plural samples, there are problems that instruments become large and cost is high. In this way, when applying an instrumental analysis to environmental monitoring, there are many problems ["Biosensing" by Ikuo Karube (1988) published by Keigaku Shuppan]. For such problems, biosensors are receiving public attention in recent years and studies have been conducted therefor.

A biosensor stands for a device by which physiological or biochemical changes are detected, identified and recorded. The function is based on biochemical specificity of a substance having a biological activity and cells and tissues of higher animals and plants, enzymes, antibodies, DNA, receptors, cells of microbes, etc. are biological measuring elements. As in the examples such as enzyme vs. substrate, antibody vs. antigen and receptor vs. ligand, specificity of the reaction is a main advantage of a biosensor. Additional advantages of a biosensor are that it has a sensitivity keenly responding even to a substance in small amount, that it achieves the function even in a non-transparent solution unlike the optical instrumental analysis means, that a direct measurement is possible whereby separation analysis and purification of sample are not necessary, that the system is able to be made small due to the above whereby conveyance is good, that powerful chemicals and hazardous reagents are unnecessary since it is derived from biological element and monitoring is possible under a mild environment of ambient temperature and pressure, that a real-time analysis is possible due to quick response and that operation is simple ["Biosensing" by Ikuo Karube (1988) published by Keigaku Shuppan; S.F.D'Souza, (2001) Biosens.Bioelectron., 16:337-53].

Incidentally, in elements of a biosensor, enzymes and antibodies have very high specificity and are useful but, on the other hand, there are disadvantages that purification thereof needs much time and high cost and that inactivation due to instability of the molecule itself and to toxic substance in environment such as heavy metal is apt to happen. In view of environmental monitoring, a microbial biosensor is believed to be advantageous as compared with other types of biosensor due to the characteristics that genetic operation such as mutation and recombination is easy, that cost is able to be reduced because incubation is easy, that there are microbes suitable even for severe environmental condition due to acquirement of resistance, etc.

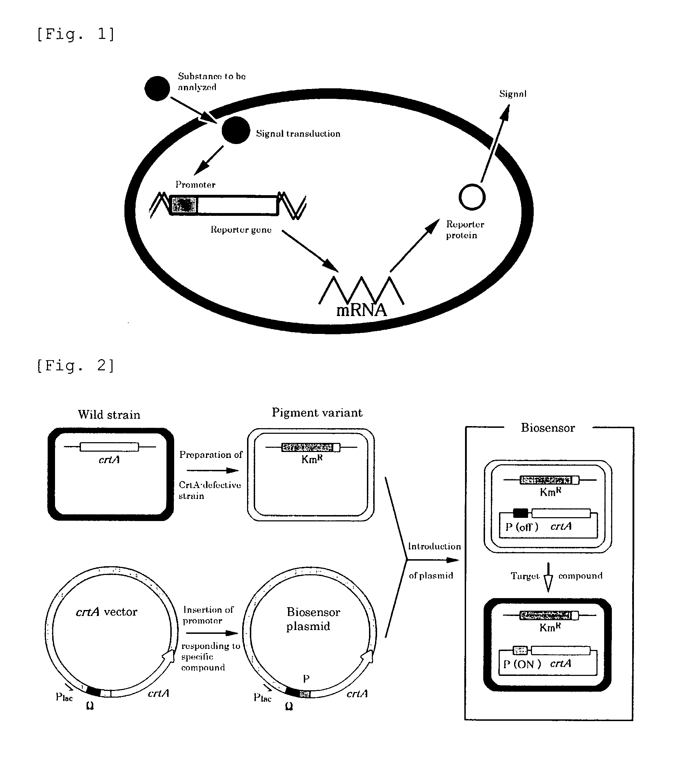

Microbial biosensors fundamentally comprise the following elements, i.e. a recombinant microbe as a host, a gene promoter sequence specifically responding to a specific chemical substance and a reporter gene located at downstream region of the promoter sequence and, in some cases, a physico-chemical device necessary for detecting the signal from the sensor is combined therewith (Fig. 1, [S.Belkin, (2003) Cur.Opin.Microbiol., 6:206-12]). When environmental stress or substance to be analyzed is present, its signal is transmitted into the microbe cells in such a system whereby the function of the promoter sequence becomes specifically "on" and gene transfer at the downstream region of the promoter sequence is induced. By detecting and measuring the expression signal of the reporter gene, an environmental monitoring is possible. When a microbial biosensor which has been actually conducted up to present is taken as an example, representative ones are that, with regard to a promoter, binary control system or repressor which is originally owned by a host such as heavy metal-responding promoter or DNA damage-responding promoter is mediated for response regulation and that, with regard to reporter gene, the gene which is able to detect and measure the signal with high sensitivity such as fluorescent protein or gene participating in luminescence is used [S.F.D' Souza, (2001) Biosens.Bioelectron., 16:337-53].

Particularly with regard to reporter gene, various comparative investigations have been carried out already and problems for each of them have been found as well [K.Hakkila, et al., (2002) Anal.Biochem., 301:235-42]. In green fluorescent protein (GFP) and red fluorescent protein (DsRed), protein itself expressed from each gene emits fluorescence without addition of co-factor, substrate, etc. whereby simple measurement and high sensitivity can be expected but they have a disadvantage that long time is needed until the translated protein is transformed into an active type where its conformation is able to emit fluorescence. Although lucFF which is firefly luciferase gene and luxCDABE which is microbioluminescence gene operon are also reporter genes which are frequently used for microbial biosensors, the former is required that a reaction substrate is added thereto while, in the latter, it codes for enzyme which produces a substrate of luminous reaction and enzyme which catalyzes the luminous reaction whereby there is no need of adding the substrate, etc. although, the fact that bacteria cells are always exposed to optical stress and that, since production of five polypeptides is inevitable, load is applied to cell growth and metabolism depending upon copy numbers of plasmid having reporter gene has been pointed out. In addition, in both luminous and fluorescent reporter genes, measuring requirements such as light-resistant chamber, optical fiber and high-sensitivity photometer are necessary for the measurement of a sample.

Among the above-mentioned hazardous substance polluting the aqueous environment, a particularly serious one is arsenic. Although its concentration is as very low as 0.0001%, arsenic is widely distributed on the surface of the earth and is present by bonding to minerals of metals such as copper, lead and gold. Most of arsenic is eluted into a hydrosphere by action of rivers and rain and accumulated in underground water or marine water [R.S.Oremland, et al., (2003) Science 300:939-44]. As such, arsenic has a characteristic that it is accumulated in a specific hydrosphere from the geological point of view. However, accumulation of arsenic affects a serious influence on the health of persons living the area around there and causes health troubles such as arteriosclerosis, myocardial infarction and peripheral circulatory troubles of the limbs. At present, the areas where severe affection by arsenic is noted are found throughout the world. Places where many patients suffering from arsenic intoxication are noted are whole area of Bangladesh, West Bengal areas of India and Inner Mongolia Autonomous Region of China and, besides them, presence of patients suffering from arsenic intoxication has been also confirmed in the State of Códoba of Argentina and Torreón of Mexico. Most of the causes thereof are use of underground water polluted with arsenic. A fundamental solving means therefor is to secure water source which is not polluted with arsenic but, due to the problem of economic circumstance, etc. of such areas, the current status is that it has not been solved yet.

On the other hand, detection of arsenic has been usually conducted by means of an inductively coupled plasma emission spectrometry (ICP-AES) where a measurement with high sensitivity is possible. However, the apparatus used for this analytical method is very expensive (several ten million yens) whereby an enormous cost is needed. Therefore, relatively less expensive (but less sensitive) kits for the detection of arsenic which utilize chemical reaction have been developed and actually used. In such a detection kit however, an operation process is troublesome and powerful chemicals such as hydrochloric acid or tin chloride are needed for the reaction and, therefore, there is a problem that arsenic is unable to be measured easily [M.M.Rahman, et al., (2002) Environ.Sci.Technol., 36:5385-94].

### Disclosure of the Invention

### Problems that the Invention is to Solve

The present invention has been created in view of the above-mentioned current status of the prior art and its object is to provide a biosensor where measurement is able to be conducted by simpler operations, cost is less expensive and specific chemical substance is able to be visually detected. Another object of the present invention is to provide an apparatus by which arsenic is able to be detected in low cost, with high sensitivity and by easy operations.

### Means for Solving the Problems

In order to achieve such objects, the present inventors have carried out intensive studies paying their attention to pigment synthesis system of purple non-sulfur bacteria belonging to the family of photosynthetic bacteria and, as a result, they have found that enzyme gene carrying a leading role in the pigment synthesis system of this bacterium is very useful as a reporter gene of a biosensor whereupon the present invention has been achieved. The present inventors have also thought that arsenic is able to be detected in low cost, with high sensitivity and by simple operations when an inductive promoter showing a specific response to arsenic is adopted as an inductive promoter for the biosensor and have carried out intensive studies for an appropriate arsenic-inductive promoter sequence and, as a result, they have achieved the present invention.

Thus, in accordance with the present invention, there is provided a biosensor for the detection of a specific chemical substance, characterized in that it comprises a recombinant purple non-sulfur bacterium wherein sensor vector is introduced into a pigment variant of a purple non-sulfur bacterium being deficient in gene coding for a spheroidene monooxygenase enzyme, and that the sensor vector is a sensor vector where inductive promoter showing a specific response to a specific chemical substance is connected in an operable manner to the upstream region of gene coding for the spheroidene monooxygenase enzyme.

In accordance with the present invention, there is also provided a method for the preparation of a biosensor for detection of a specific chemical substance, characterized in that it comprises the steps of:
preparing a pigment variant of a purple non-sulfur bacterium being deficient in gene coding for spheroidene monooxygenase enzyme;
cloning an open reading frame of gene coding for spheroidene monooxygenase enzyme being deficient in promoter on vector;
connecting an inductive promoter showing a specific response to a specific chemical substance in an operable manner to the upstream region of gene coding for spheroidene monooxygenase enzyme in the vector to construct a sensor vector; and
introducing the sensor vector into the pigment variant of a purple non-sulfur bacterium to prepare a recombinant purple non-sulfur bacterium.

According to preferred embodiment of the present invention, the above-mentioned purple non-sulfur bacterium is *Rhodovulum sulfidophilum.*

According to preferred embodiment of the present invention, the above-mentioned specific chemical substance is dimethyl sulfide and the inductive promoter showing a response to the above-mentioned specific chemical substance is dimethyl sulfide-inductive promoter.

According to more preferred embodiment of the present invention, the above-mentioned dimethyl sulfide-inductive promoter is a promoter which is present in an upstream region of operon of gene coding for dimethyl sulfide dehydrogenase enzyme.

According to the particularly preferred embodiment of the present invention, the above-mentioned promoter is DNA consisting of a base sequence of SEQ ID No. 1 or DNA consisting of a base sequence which is at least 60% homologous to the base sequence of SEQ ID No. 1 and having an activity of dimethyl sulfide-inductive promoter.

According to another preferred embodiment of the present invention, the above-mentioned specific chemical substance is arsenic and the inductive promoter showing a response to the above-mentioned specific chemical substance is DNA consisting of a base sequence of SEQ ID No. 29 or DNA consisting of a base sequence which is at least 60% homologous to the base sequence of SEQ ID No. 29 and having an activity of arsenic-inductive promoter.

### Advantages of the Invention

In the biosensor of the present invention, gene coding for spheroidene monooxygenase enzyme is used as a novel reporter gene as mentioned above whereby the measured result is able to be expressed as a clear color tone change of the culture liquid of the biosensor. Accordingly, when the biosensor of the present invention is used, no measuring equipment such as a highly sensitive photometer in the case of biosensors in the prior art using luminous or fluorescent reporter gene is necessary and the hazardous substances in environment are able to be visually detected by a simple means. Particularly, the biosensor in the preferred embodiment of the present invention adopts an inductive promoter showing a specific response to arsenic as an inductive promoter of the biosensor whereby it is now possible to detect arsenic in low cost, with high sensitivity and by a simple operation.

### Brief Description of the Drawings

Fig. 1 shows a principle of a microbial biosensor.
Fig. 2 shows a construction process of a visual biosensor. In the drawing, P is a promoter, Ω is a terminator hairpin structure and Km^{R} is a kanamycin-resisting gene cassette.
Fig. 3 shows a construction process of an arsenic-detecting biosensor of the present invention. In the drawing, P is a promoter.
Fig. 4 shows a principle of the arsenic-detecting biosensor of the present invention.
Fig. 5 shows HPLC eluting profiles of pigment extracted from *Rv. sulfidophilum* W-1S strain (A) and from crtA⁻ strain (B). Several peaks shown by numerals are referred to in the text.
Fig. 6 shows absorption spectrum of the pigment separated by an HPLC. Each sample is shown in Fig. 5.
Fig. 7a shows LC-MS spectrum of a carotenoid pigment. Peak numbers of each sample correspond to Fig. 5.
Fig. 7b shows LC-MS spectrum of a carotenoid pigment. Peak numbers of each sample correspond to Fig. 5.
Fig. 8 shows alignments of CrtA amino acid sequence (Rb. cap.) of *Rb*. *capsulatus,* CrtA amino acid sequence (Rb. sph.) of *Rb*. *sphaeroides* and a putative amino acid sequence (Rv. sul.) of *Rv*. *sulfidophilum.*
   In Fig. 9, (A) shows, by arrows, hybridized positions of primers (U1, crtAup1; U2, crtAup2; D1, crtAdown1; D2, crtAdown2) used for amplification of fragment U and fragment D while (B) shows construction of plasmid for cloning of Fragment UKD.
Fig. 10 shows amplification of DNA fragment including crtA gene and upstream and downstream regions thereof by PCR using primers crtAup1 and crtAdown2. Annealing temperature is shown above each lane. M is a mixture of 100-bp DNA ladder and λ-HindIII marker.
Fig. 11 shows amplification of DNA fragment by PCR. Lane D is a PCR using primers crtAup1 and crtAup2; lane U is a PCR using primers crtAdown1 and crtAdown2; and M is a mixture of 100-bp DNA ladder and λ-HindIII marker.
Fig. 12 shows amplification of DNA fragment by a PCR for checking the Fragment UKD using three primer pairs. The primer pair a is crtAup1 and crtAup2, b is crtAdown1 and crtAdown2 and c is crtAup1 and crtAdown2.
Fig. 13 shows color tone of wild strain (three in the left) and pigment variant (three in the right).
Fig. 14 shows amplification of DNA fragment by a PCR using a primer where crtA gene is a target. Lane M is λ-EcoT14I, lanes 32 to 34 are conjugants, lane S is a single-crossed pigment variant, lane W is *Rv. sulfidophilum* W-1S strain and lane E is *E. coli* S17-1 λ-pir having a plasmid pHDR2.
Fig. 15 shows HPLC eluting profiles of carotenoid from *Rv. sulfidophilum* strains crtA⁻32, crtA⁻33 and crtA⁻34. DMSE is demethylspheroidene, SO is spheroidenone and SE is spheroidene.
Fig. 16 shows a possible structure for a terminator sequence Ω.
Fig. 17 shows maps of pRV1Ω and pRV5Ω. Tc^{R} is a tetracycline-resisting gene, SD is a Shine-Dalgarno sequence, Plac is lacZ promoter, Pcrt is crtA promoter and Ω is a terminator. An arrow shows the direction of transcription.
Fig. 18 shows HPLC elution profiles of carotenoid from *Rv. sulfidophilum* strain W-1S, crtA 32 and transformants thereof (pRV1Ω and pRV5Ω). DMSO is demethylspheroidenone, DMSE is demethylspheroidene, SO is spheroidenone, SE is spheroidene and NS is neurosporene.
Fig. 19 shows amplification of DNA fragment by a PCR using primer where ddhA gene and rRNA are targets. Annealing temperature is shown on each lane.
Fig. 20 shows transcription induction of ddhA by addition of DMS under various growing conditions.
Fig. 21 shows an inverse PCR means by a schematic way.
Fig. 22 shows *Rv. sulfidophilum* W-1S strain genomic DNA subjected to a treatment with restriction enzyme by SalI. DNA fragment contained in each fraction was extracted.
Fig. 23 shows amplification of ddhA gene fraction by a PCR for detection of fraction containing ddhA region.
Fig. 24 shows application of an inverse PCR for amplification of flanking region of ddhA gene. Template DNA was extracted from 3 to 4-kb fractions.
Fig. 25 shows DNA sequence of upstream region of ddhA.
Fig. 26 shows construction of sensor plasmid for detection of DMS.
Fig. 27 shows HPLC elution profile of carotenoid from DMS biosensor of *Rv. sulfidophilum* strain. Bchl is bacteriochlorophyll, DMSE is demethylspheroidene, SO is spheroidenone and SE is spheroidene.
Fig. 28 shows response of DMS biosensor to DMS on the third day of incubation.
Fig. 29 shows response of DMS biosensor to DMS on the sixth day of incubation.
Fig. 30 shows a PCR program using Example 3.
Fig. 31 shows the result of homologous gene investigation of E. *coli* arsR in *Rv. sulfidophilum* by a southern hybridization analysis. Hybridization temperatures are 50°C (A), 55°C (B) and 60°C (C). Lane 1: digested product with EcoRI; lane 2: digested product with HindIII; lane 3: digested product with PstI; lane 4: digested product with SacI; and lane 5: E. *coli* genomic DNA digested by PvuII as a positive control.
Fig. 32 shows construction of pRV5Ω Pars for evaluation of promoter activity of E. *coli* Pars. In the drawing, P means Pars.
Fig. 33 shows the color of a crtA⁻32 transformant pRV5Ω Pars.
Fig. 34 shows HPLC chromatographic profiles of carotenoid from the crtA⁻32 transformants (pRV5Ω, pRV5Q Pars and pRV1Ω). DMSO: demethylspheroidenone; DMSE: demethylspheroidene; SO: spheroidenone; SE: spheroidene.
Fig. 35 shows E. *coli* Pars and arsR as control sequences of crtA.
Fig. 36 shows construction of pSENS-AS strain as a biosensor plasmid for detection of arsenic.
Fig. 37 shows response of SENS-AS strain to As₂O₃ after incubation for 24 hours.
Fig. 38 shows a relative value of total carotenoid in 1 ml of a culture liquid.
Fig. 39 shows transcription induction of crtA and arsR by addition of 0.06 ppm of As₂O₃.
Fig. 40 shows the optimum reaction time.
Fig. 41 shows specificity of SENS-AS strain. As: arsenic; Bi: bismuth; Cd: cadmium; Co: cobalt; Ni: nickel; Pb: lead; Sb: antimony; Zn: zinc; Mix: Fe + Co + aluminum + manganese. Best Mode for Carrying Out the Invention

A purple non-sulfur bacterium which is the main constituting element of the biosensor of the present invention synthesizes carotenoid pigment from geranylgeranyl diphosphate which is a common product of isoprenoid biosynthesis route. In the freshwater genus Rhodobacter which is a type of purple non-sulfur bacterium, it has been already known that eight kinds of enzymes concerning carotenoid synthesis route are present and that genes coding therefor form cluster [H.P.Lang, et al., (1995) J.Bacteriol., 177(8):2064-73]. A purple non-sulfur bacterium accumulates spheroidenone which is the final product of carotenoid or a derivative thereof and the culture liquid shows reddish brown color derived from the pigment.

Production of spheroidenone is carried out by oxidation of spheroidene which is a precursor yellow pigment (or a derivative thereof) and this reaction is catalyzed by CrtA enzyme (spheroidene monooxygenase). Accordingly, in a purple non-sulfur bacterium strain where CrtA enzyme is deficient, the culture liquid turns yellowish brown as a result of accumulation of spheroidene. The idea which exists in the base of the present invention is that, as a result of regulation of the expression of this CrtA enzyme, changes in color tone of the culture liquid from yellow to red are achieved and, as a result of its utilization, construction of "visual biosensor" is made possible. Particularly, the idea which exists in the base of the present invention is that, as a result of regulation of expression of this CrtA enzyme using an arsenic-inductive promoter, changes in color tone of culture liquid from yellow to red are achieved only in the presence of arsenic whereby presence of arsenic is visually detected.

Flow of construction of the "visual biosensor" of the present invention is in accordance with the process as shown in Fig. 2.
Firstly, in a purple non-sulfur bacterium, there is constructed a pigment variant which is a variant where gene (crtA) coding for CrtA is deficient and yellow color is resulted as spheroidene is accumulated. After that, there is constructed a crtA vector where crtA gene ORF being deficient in promoter sequence is cloned on plasmid. The so-called inductive promoter showing a specific response to a specific chemical substance is inserted into the crtA vector whereupon a sensor plasmid is constructed. Finally, the sensor plasmid is introduced into the pigment variant to construct the aimed biosensor strain. In the biosensor strain constructed as such, the inductive promoter in the upstream region of the crtA gene does not work in the absence of the target compound and spheroidene is accumulated due to deficiency of CrtA to give yellowish brown color. On the contrary, in the presence of the target compound, the inductive promoter is activated and transcription of the crtA gene arranged in the downstream region is promoted. The accumulated spheroidene is converted to spherdoidenone by the expressed CrtA and the cells turn reddish brown color. As a result of the changes in color tone as such, detection of the target compound is now possible.

With regard to the purple non-sulfur bacterium used for the biosensor of the present invention, any species may be used so far as it is a purple non-sulfur bacterium which has gene coding for spheroidene monooxygenase enzyme acting as a reporter gene in the biosensor in genome and has an ability of producing reddish brown pigment spheroidenone by oxidation of yellow pigment spheroidene and of accumulating it in the bacterium cell and, for example, *Rhodovulum sulfidophilum* which is a marine purple non-sulfur bacterium is able to be used. As to the *R. sulfidophilum,* cloning of gene crtA coding for spheroidene monooxygenase enzyme and decoding of its base sequence have been completed already in the previous study by the present inventors. Accordingly, *R. sulfidophilum* is convenient for using as a purple non-sulfur bacterium containing reporter gene.

In the biosensor of the present invention, the above-mentioned purple non-sulfur bacterium is not used as it is but a pigment variant where gene coding for a spheroidene monooxygenase is deficient is prepared and, into this pigment variant, a sensor vector in which an inductive promoter specifically responding to a specific chemical substance which is an object to be detected by the biosensor is connected, in an operable manner, to the upstream region of reporter gene by means of a genetic engineering technique is introduced and the recombinant prepared thereby is used as a biosensor. With regard to an inductive promoter which specifically responds to a specific chemical substance, that which is appropriate corresponding to the object to be detected by the biosensor of the present invention may be appropriately selected and, when the object to be detected by the sensor is dimethyl sulfide for example, it is possible to use a promoter which is present in the upstream region of operon of gene coding for a dimethyl sulfide dehydrogenase enzyme as a dimethyl sulfide-inductive promoter showing a specific response to dimethyl sulfide. To be more specific, this promoter is DNA consisting of a base sequence of SEQ ID No. 1. It is also possible to use DNA consisting of the base sequence having at least 60% of homology to the base sequence of SEQ ID No. 1 and having a dimethyl sulfide-inductive promoter activity or, in other words, DNA within an equivalent range to the base sequence of SEQ ID No. 1 as a promoter in the biosensor of the present invention.

Preferred range of homology of the DNA within an equivalent range to the base sequence of SEQ ID No. 1 is at least 70%, more preferably at least 80%, still more preferably at least 90%, further preferably at least 95% and, most preferably, at least 98%. The homologous DNA as such is able to be easily prepared by, for example, a well-known site-specific mutagenesis (Nucleic Acid Research, Vol.10, No.20, p.6487-6500,1982). Further, the fact whether the resulting DNA has a dimethyl sulfide-inductive promoter activity is able to be confirmed, for example, by such a manner that a transformed *Escherichia coli* into which the resulting DNA is introduced into an upstream region of an appropriate reporter gene is prepared and incubated in the presence of dimethyl sulfide to check whether the reporter gene is expressed.

In the present invention, the term reading "connected in an operable manner" means that a promoter is arranged on the upstream region of the gene in such a manner that expression control of gene is made possible.

The object to be detected by the biosensor of the present invention is determined by the specificity of the inductive promoter used and any chemical substance may be used as an object to be detected so far as an inductive promoter showing a specific response to the target chemical substance is present. The biosensor of the present invention is able to be used particularly advantageously for the detection of agricultural chemicals, heavy metals and endocrine disrupting chemicals in aqueous environments such as the ocean, rivers and lakes utilizing its simplicity for measuring operations and visibility of the measured results. When, for example, a dimethyl sulfide-inductive promoter is selected as an inductive promoter, the biosensor of the present invention is able to be used in the detection of dimethyl sulfoxide in the ocean which has a risk of contributing in climate change of the earth and to investigate the changes of its concentration.

In the biosensor of the present invention, a constitutive promoter which is inherently present in an upstream region of gene coding for a spheroidene monooxygenase enzyme of a purple non-sulfur bacterium is substituted with an inductive promoter which specifically responds to a specific chemical substance by some way and, to be more specific, it is able to be prepared by the following steps. Thus:
gene coding for a spheroidene monooxygenase enzyme is substituted with other gene such as, for example, kanamycin-resisting gene to prepare a pigment variant of non-sulfur bacterium where gene coding for spheroidene monooxygenase enzyme is deficient;
an open reading frame of gene coding for a spheroidene monooxygenase enzyme being deficient in promoter is cloned onto a vector such as plasmid;
an inductive promoter specifically responding to a specific chemical substance is connected in an operable manner to the upstream region of gene coding for a spheroidene monooxygenase enzyme in the vector to construct a sensor vector; and
the sensor vector is introduced into a pigment variant of a purple non-sulfur bacterium to prepare a recombinant purple non-sulfur bacterium.
All of those steps may be appropriately carried out by common methods which have been known well by persons skilled in the art and detailed illustration therefor will be omitted.

In order to detect the chemical substance to be detected using the biosensor of the present invention, the recombinant purple non-sulfur bacterium constituting the biosensor of the present invention is incubated under a photoaerobic condition, a sample having a possibility of containing the chemical substance to be detected is added thereto and incubation is further continued. If no chemical substance to be detected is contained in the sample, the inductive promoter still remains inactive whereby the purple non-sulfur bacterium cell is unable to transcribe/express the spheroidene monooxygenase enzyme gene and, due to the deficiency of the enzyme, a yellow pigment spheroidene is accumulated in the cell and the color still remains yellow brown. On the contrary, when a chemical substance to be detected is contained in the sample, the inductive promoter is activated, transcription of the spheroidene monooxygenase enzyme gene existing in the downstream region is promoted, the spheroidene monooxygenase enzyme is expressed in the purple non-sulfur bacterium cell, the yellow pigment spheroidene accumulated in the cell is oxidized due to the catalytic action of the enzyme so that it is converted to a reddish brown pigment spheroidenone and the color tone of the cell turns from yellowish brown to reddish brown. As a result of the color tone change as such, a visual detection of the chemical substance to be detected is now possible.

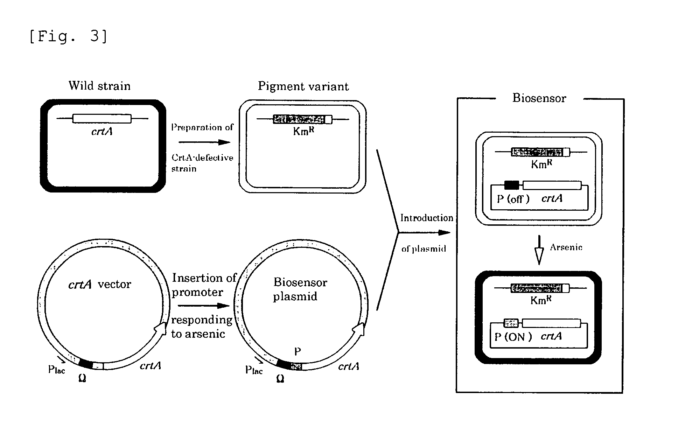

Now the biosensor for detection of arsenic which is the particularly preferred embodiment of the present invention will be illustrated as hereunder.
Flow of the construction of the biosensor for detection of arsenic by the present invention is in accordance with the process as shown in Fig. 3.
Firstly, there is constructed a pigment variant which is a variant being deficient in gene (crtA) coding for CrtA in the purple non-sulfur bacterium and showing yellow color as spheroidene is accumulated. After that, there is constructed a crtA vector in which crtA gene ORF being deficient in promoter sequence is cloned onto a plasmid. When the so-called inductive promoter showing a specific response to arsenic is inserted into the crtA vector, a sensor plasmid is constructed. Finally, the sensor plasmid is introduced into the pigment variant whereupon the aimed biosensor strain is constructed. In the biosensor strain constructed as such, the inductive promoter in the upstream region of the crtA gene in the absence of arsenic does not work and spheroidene is accumulated due to deficiency of CrtA to give yellowish brown color. On the other hand, in the presence of arsenic, the inductive promoter is activated and transcription of crtA gene arranged in the downstream region is promoted. Due to the expressed CrtA, the accumulated spheroidene is converted to spheroidenone and the cells turn reddish brown. As a result of the changes in color tone as such, detection of arsenic is possible.

The arsenic-inductive promoter used in the biosensor for the detection of arsenic according to the present invention consists of a promoter region of upstream region of arsenic-resisting gene operon of *Escherichia coli* and gene region of the most upstream region of arsenic-resisting gene operon. The arsenic-resisting gene operon of E. *coli* is constituted from three genes - arsR, arsB and arsC - and Pars which is a promoter region is ligated to the upstream region of arsR gene which is the most upstream region. This operon is characterized in being controlled that, only when arsenic is present, it is strongly expressed and this control is carried by an interaction of ArsR which is an expression control protein for which arsR gene is coded with Pars which is a promoter region of arsRBC. ArsR protein has a sequence showing a bonding ability to arsenic and a sequence having a bonding ability to Pars which is a promoter region of arsRBC and, when no arsenic is present, it is bonded to Pars to suppress the transcription of arsRBC. On the other hand, when arsenic is present, ArsR protein is bonded to arsenic, causes a conformation change and is dissociated from Pars whereupon transcription from Pars takes place. As such, in the arsenic-resisting gene operon of *E. coli,* response to arsenic and control of transcription are carried out due to an interaction of ArsR protein with Pars which is a promoter region of arsRBC. In the present invention, the Pars and arsR regions of *E*. *coli* as such are used as a control system of the biosensor whereby construction of biosensor which is able to detect arsenic is made possible.

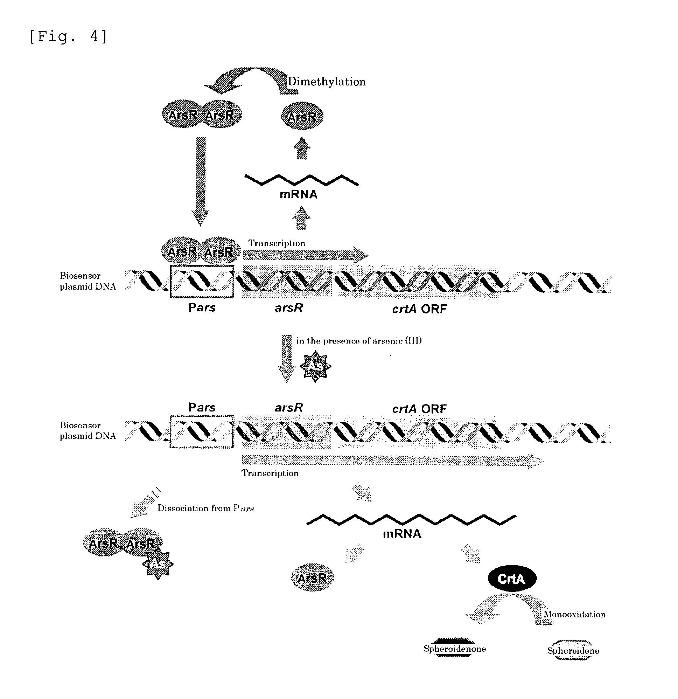

Now the principle of detection of arsenic using the arsenic-detecting biosensor of the present invention will be illustrated in more details by referring to Fig. 4. In order to detect arsenic using the arsenic-detective biosensor of the present invention, the recombinant purple non-sulfur bacterium constituting the arsenic-detecting biosensor of the present invention is incubated under a bright aerobic condition, a sample having a possibility of containing arsenic is added to the medium and incubation is further continued. When no arsenic is contained in the sample, the inductive promoter remains inactive since the Pars region of the inductive promoter is bonded to ArsR protein (in a state of a dimer) and the purple non-sulfur bacterium cell is unable to transcribe/express the spheroidene monooxygenase enzyme gene whereby, due to deficiency of the enzyme, the yellow pigment spheroidene is accumulated in the cell and the yellowish brown color still remains (refer to the upper side of Fig. 4). On the contrary, when arsenic is contained in the sample, ArsR protein is bonded to arsenic to cause a change in conformation and is dissociated from the Pars region and, as a result, the inductive promoter is activated, transcription of a spheroidene monooxygenase enzyme gene existing in the downstream region is promoted and spheroidene monooxygenase enzyme is expressed in the purple non-sulfur bacterium cell whereupon, due to the catalytic action of this enzyme, the yellow pigment spheroidene accumulated in the cell is oxidized to be converted into a reddish brown pigment spheroidenone and the color tone of the cell changes from yellowish brown to reddish brown (refer to the lower side of Fig. 4) . As a result of changes in color tone as such, detection of arsenic is now possible.

To be more specific, the arsenic-inductive promoter which is able to be used in the arsenic-detecting biosensor of the present invention is DNA consisting of the base sequence of SEQ ID No. 29. In addition, DNA consisting of the base sequence which is at least 60% homologous to the base sequence of SEQ ID No. 29 and having an arsenic-inductive promoter activity or, in other words, DNA which is within an equivalent range to the base sequence of SEQ ID No. 29 is also able to be used as an arsenic-inductive promoter in the arsenic-detecting biosensor of the present invention.

Preferred range of homology of DNA within an equivalent range to the base sequence of SEQ ID No. 29 is at least 70%, more preferred range is at least 80%, still more preferred range is at least 90%, further preferred range is at least 95% and the most preferred range is at least 98%. Incidentally, such a homologous DNA may be easily prepared by, for example, a well-known site-specific mutagenesis [Nucleic Acid Research,Vol.10,No.20,p.6487-6500,1982]. The fact that whether the resulting DNA has an arsenic-inductive promoter activity is able to be confirmed by such a manner, for example, that a transformant *E*. *coli* where the resulting DNA is introduced into an upstream region of an appropriate reporter gene is prepared and incubated in the presence of arsenic to check whether the reporter gene is expressed.

In the present invention, the term "arsenic-inductive promoter" or "an inductive promoter showing a specific response to arsenic" means DNA which makes, only in the presence of arsenic, transcription of gene ligated to downstream region thereof possible and that is a conception including, if necessary, a gene sequence of additional control protein which interacts to a promoter region such as arsR gene of E. *coli* in addition to the common promoter region.

### Examples

The present invention will now be specifically illustrated by way of the following Examples. Incidentally, descriptions in the Examples are purely for understanding of the invention only and the present invention is not limited thereby at all.

### Example 1

### Materials and methods for experiments

### 1: Strains and media used in this Example

Strains used in this Example are shown in Table 1 while media used therein are shown in Table 2. *Rhodovulum sulfidophilum* W-1S train has been deposited as FERM P-15320 at the International Patent Organism Depositary of the National Institute of Advanced Industrial Science and Technology [Center No. 6, 1-1, Higashi-1-chome, Tsukuba-shi, Ibaraki Prefecture, Japan (post office code: 305-8566)] (old name: the National Institute of Bioscience and Human Technology, Agency of Industrial Science and Technology, MITI [1-3, Higashi-1-chome, Tsukuba-shi, Ibaraki Prefecture, Japan (post office code: 305)] (date of deposition: December 11, 1995).
A 4CyMOM medium was used for incubation of *Rv. sulfidophilum* W-1S strain and variants and transformant strains thereof. For the incubation of the biosensor strain, 4CMOM medium was prepared as a 4CyMOM medium containing no yeast extract and then used therefor. For the incubation of E. *coli* JM109 strain, *E*. *coli* S17-1 strain, *E*. *coli* S17-1 λ-pir strain and transformant strain for each of them, an LB medium was used. If necessary, ampicillin (Amp), kanamycin (Km) or tetracycline (Tc) was added to each medium so as to make the final concentration thereof 50 µg/ml, 30 µg/ml or 20 µg/ml, respectively. With regard to a photosynthetic bacterium however, the amount of Tc to be added was adjusted so as to make its final concentration 5 µg/ml.
An SOC medium was used during a temporary incubation after electroporation and during the transformation process.

**Table 1 Strains used in this Example**

| Strain | Origin or reference documents |
|---|---|
| ***Rhodovulum sulfidophilum* W-1S** | **2** |
| ***Rhodovulum sulfidopbilum* crtA⁻1** | **1** |
| ***Escherichia coli* JM109** | **TaKaRa** |
| ***Escherichia coli* S17-1** | **3** |
| ***Escherichia coli* S17-1 λ-*pir*** | **4** |

### 2: Plasmids used in this Example

The plasmids used in this Example will be summarized later. Hereinafter, basic ones for construction of each plasmid and vector will be mentioned.
A fragment amplified by a PCR was incorporated into pGEM-T Easy Vector (Promega). For the preparation of *Rv*. *sulfidophilum* pigment variant, pSM3065 [S.Masuda, et al., (1999) J.Bacteriol., 181(14):4205-15] which is a suicide vector was utilized. For the expression of crtA in *Rv. sulfidophilum* pigment variant, pRK415 [N.T.Keen, et al., (1988) Gene, 70:191-7] which is a broad host range vector was used. With regard to a kanamycin cassette (KmR), a sequence contained in pUC4K (Amersham Pharmacia Biotech) was utilized.

**Table 2 Composition of media used in this Example**

| | | | |
|---|---|---|---|
| LB Medium | | **4CyMOM** | |
| Tryptone peptone | **10g** | **NaCl** | **30g** |
| Yeast extract | **5g** | **CaCl₂ · 2H₂O** | **0.2g** |
| **NaCl** | **10g** | **MgSO₄ · 7H₂O** | **0.25g** |
| Distilled water was added to make the total amount 1 liter | | **FeSO₄ · 7H₂O** | **0.02g** |
| | | **KH₂PO₄** | **40.8mg** |
| SOC Medium SOC Medium | | **K₂HPO₄** | **495mg** |
| | | Thiamine | **0.5mg** |
| SOB medium | **1L** | Nicotinic acid | **0.5mg** |
| **1M MgCl₂** | **10ml** | **PABA** | **0.3mg** |
| **1M MgSO₄** | **10ml** | Biotin | **0.05mg** |
| **1M** Glucose | **20ml** | Sodium succinate | **1.0g** |
| SOB Medium | | Sodium DL-malate | **1.0g** |
| | | Sodium acetate | **1.0g** |
| Tryptone peptone | **20g** | Sodium pyruvate | **1.0g** |
| Yeast extract | **5g** | Yeast extract | **1.0g** |
| **NaCl** | **0.5g** | **1M NH4Cl** | **5.0ml** |
| **250mM KCl** | **10ml** | **A'5** solution | **1.0ml** |
| 5**N NaOH** | **0.2ml** | Distilled water was added to make the total amount 1 liter | |
| Distilled water was added to make the total amount 1 liter | | Adjusted to pH 8.0 | |
| | | **A'5** solution | |
| | | **H₃BO₄** | **2.86g** |
| | | **MnCl₂ · 4H₂O** | **1.81g** |
| | | **ZnSO₄ . 7H₂O** | **0.22g** |
| | | **CuSO₄ · 5H₂O** | **0.08g** |
| | | **Na₂M0O₄** | **0.021g** |
| | | **CoCl₂ · 6H₂O** | **0.01g** |
| | | **EDTA · 2Na** | **50g** |
| | | Distilled water was added to make the total amount 1 liter | |

### 3: Extraction of total DNA

Extraction of total DNA from *Rv. sulfidophilum* W-1S strain was carried out according to the protocol of the reference document ["Illustrated Bio-Experiments, (ii) Fundamental Gene Analysis" (1995) by Hiroki Nakayama and Takahito Nishikata, published by Shujunsha].

### 4: PCR

With regard to PCR, an operation was conducted using TaKaRa LA Taq^{™} with GC Buffer (TaKaRa) according to the protocol attached thereto. With regard to a template, each total DNA or plasmid DNA was used or a direct PCR from a culture liquid or a liquid in which colonies were suspended was carried out. In a direct PCR, 50 µl of a culture liquid or a liquid where colonies were suspended in 50 µl of H₂O was heated at 95°C for 5 minutes and the supernatant liquid after a centrifugal separation was used as a template.

### 5: Recovery of DNA fragment from gel, ligation and transformation

When the fragment amplified by a PCR is cloned, it was incorporated into pGEM-T Easy Vector and then introduced into *E. coli* JM109. The method was carried out in accordance with the protocol for pGEM-T Easy Vector System. In case a plasmid DNA was introduced into *E*. *coli* S17-1, the method therefor also followed said protocol.
The DNA fragment treated with a restriction enzyme was subjected to electrophoresis on agarose gel and then recovered by an excision from the gel. In extracting the DNA from the gel fragment, that was conducted using a dialysis tube ["A Laboratory Manual - Genetic Engineering" edited by Masahiro Muramatsu, (1988), published by Maruzen]. The liquid in the dialysis tube was recovered by paying careful attention so that gel did not come therein and the DNA was recovered by extracting with PCI (phenol-chloroform-isoamyl alcohol) ["Illustrated Bio-Experiments, (i) Fundamental Molecular Biochemical Experiments" (1995) by Takahito Nishikawa, published by Shujunsha] and by precipitating with ethanol. In the operation of precipitating with ethanol, glycogen (Nakarai Tesk) was used as a co-precipitating agent for the purpose of enhancing the recovering efficiency of DNA.
When the excised DNA fragment is subjected to ligation, it was conducted using DNA Ligation Kit Ver. 2 (TaKaRa).

### 6: Extraction of plasmid DNA

Extraction of plasmid DNA was carried out using CONCERT Rapid Plasmid Miniprep System (GIBCO BRL), QIAprep Spin Miniprep Kit (QIAGEN) or QIAGEN Plasmid Midi Kit (QIAGEN).

### 7: Conjugation: Conjugation was carried out upon introduction of the plasmid DNA into Rv. sulfidophilum from Escherichia coli into which a recombinant plasmid was introduced. The method therefor is as shown below. Incidentally, all of the things used in the following operation were subjected to sterilization using an autoclave.

*E. coli* was subjected to a shake culture at 37°C in 10 ml of an LB medium in a test tube while *Rv. sulfidophilum* was incubated under a bright condition by stirring with a stirrer in 30 ml of 4CyMOM medium in a Roux bottle. *E. coli* was incubated until OD₆₆₀ became nearly 0.2 and *Rv*. *sulfidophilum* was incubated until OD₆₆₀ became nearly 1.6 and then 170 µl and 60 µl, respectively, of each microbe liquid was mixed in an Eppendorf tube. After collecting the cells, they were suspended again in 230 µl of an LB medium and the resulting liquid was spread on an MF-Millipore Type HA Membrane Filter (Millipore) on 2% NaCl LB agar plate. After the plate was allowed to stand overnight at 32°C, the membrane was recovered into a vial. An LB medium (250 µl) was added into the vial and subjected to vortex whereupon the cells on the membrane were recovered and transferred into an Eppendorf tube. To the LB suspension was added 50 µl of T4 phage liquid [Institute for Fermentation, Osaka, (1992) Microorganisms, 9th edition:229] which was sterilized by filtration followed by incubating for 15 minutes at 37°C. The cells were collected, washed with 4CyMOM and suspended again in 4CyMOM. The cell liquid was applied on 4CyMOM agar plate and incubated for several days under a bright condition.

### 8: Electroporation

When a plasmid was introduced into *E. coli* S17-1 λ-pir, electroporation was used. A plasmid solution (2 µl) was added to 50 µl of *E. coli* S17-1 λ-pir melted on ice, mixed by means of pipetting, transferred to a cuvette and allowed to stand on ice for 10 minutes. Condition for the electroporation was as follows. Thus, voltage was 2.5 kV, gap between cuvette walls was 0.2 cm and condenser value was 1 µF. Immediately after the electroporation, 200 µl of an SOC medium was added thereto, mixed by means of pipetting, transferred to an Eppendorf tube and subjected to a shake culture at 37°C for 1 hour. After that, it was sown on an LB agar plate.

### 9: DNA sequence

For decoding of a base sequence of the fragment cloned on the pGEM-T Easy Vector, there were used Fluorescein-Labeled Primer RV-M and M4 (TaKaRa) and ABI PRISM BigDye Terminator Cycle Sequencing Ready Reaction Kit With AmpliTaq DNA Polymerase, FS (Applied Biosystems) and Sequence ABI PRISM 310 Genetic Analyzer (Applied Biosystems). Preparation of the sample was carried out according to a protocol attached to the kit while operation of the sequencer was carried out according to the manual attached to the instrument.

### 10: Pigment analysis

Composition analysis of various carotenoid pigments of wild strain, variant strain and transformant strain of *Rv. sulfidophilum* was carried out by the following method.
The bacteria strain was incubated for one night together with stirring by a magnetic stirrer under slightly aerobic and bright condition in a Roux bottle in which 30 ml of medium was placed. After a centrifugal separation of 8,000 rpm for 15 minutes, the supernatant liquid was discarded, cell pellets were frozen at -80°C for one night and, form the next day, the cells were freeze-dried for two nights. The resulting freeze-dried product was transferred from a centrifugal tube to a vial, 5 ml of a 7:2 (v/v) mixture of acetone and methanol was added thereto and the mixture was subjected to vortex for two minutes [S.Takaichi, et al., (2001) Photosynth.Res., 67:207-14] . The suspension was filtered and used as a sample for a high-performance liquid chromatography (HPLC).
When incubation of a bacterium strain was conducted in 10 ml of medium in a test tube under various conditions (under slightly aerobic and bright condition; under slightly aerobic and dark condition; and under anaerobic and bright condition), the incubation was done for three days. The culture liquid (1 ml) was placed in an Eppendorf tube, the pellets after centrifugal separation was frozen at -80°C for 2 hours and then freeze-dried for one night. An appropriate amount (100 µl to 500 µl) of a mixed liquid of acetone and methanol was added to the resulting freeze-dried product, subjected to vortex for 2 minutes and then centrifuged for 5 minutes at 14,000 rpm using a portable centrifuge and the resulting supernatant liquid was used as a sample for an HPLC.
The HPLC analysis was carried out using instruments for high-performance liquid chromatography manufactured by Shimadzu, liquid-sending unit LC-10AS, autoinjector SIL-9A, detector for spectrophotometer for ultraviolet and visible regions SPD-10A and chromatopack C-R3A (Shimadzu) and µ Bondapack TM C18 125Å 10 µm 3.9 x 300 mm HPLC column (Waters). Methanol was used for a liquid phase and the detection was conducted with a measuring wavelength of 481 nm. Infusing amount of the sample, flow rate of the liquid phase, recording condition for chromatopack, etc. were appropriately modified depending upon the conditions such as sample concentration.

### 11: RNA extraction and RT-PCR

Extraction of RNA was carried out using RNeasy Mini Kit (QIAGEN) according to the protocol attached thereto. For dissolving the cells at that time, lysozyme was added thereto in the indicated concentration. For the removal of the contaminated DNA, DNase I (TaKaRa) was used by the method indicated in the protocol together with the buffer attached thereto. Incubation was carried out at 37°C for 30 minutes and, after that, RNA was purified via a PCI treatment and a precipitating operation with ethanol. A reverse transcription reaction was carried out where 1 µg of purified RNA as a template using an RNA PCR Kit (AMV) Ver. 2.1 (TaKaRa). In the reverse transcription reaction, things contained in the kit, i.e. reverse transcriptase, RNase inhibitor, random nonamer, buffer, dNTP and MgCl₂ were used and the reaction was conducted at 45°C for 28 minutes. A PCR was carried out by the same manner as in the above mentioned "4: PCR" using 4 µl of the solution after the reverse transcription reaction as a template.

Example 1-A Analysis of pigment composition of *Rv*. *sulfidophilum*

### 1. Object of the Example

The present inventors have already succeeded in constructing a CrtA-deficient pigment variant crtA⁻1 in which deficiency in CrtA is believed to be resulted due to insertion of kanamycin-resisting gene cassette (Km^{R}) into crtA sequence on genome of *Rv. sulfidophilum* W-1S. However, although this pigment variant initially showed yellow color in color tone of culture liquid as predicted, it was found to gradually get tinged with red as the incubation was continued. With an object of obtaining basic data such as pigment composition of *Rv*. *sulfidophilum* including analysis for the above-mentioned point, analysis of pigment composition via pigment extraction and HPLC was conducted here. Incidentally, it is important to find the changes in carotenoid pigment composition using an HPLC for detailed comparison and analysis of state of expression of CrtA in various variants prepared in each stage of development of biosensors such as pigment variants and transformant strains thereof.

### 2. Pigment analysis of Rv. sulfidophilum

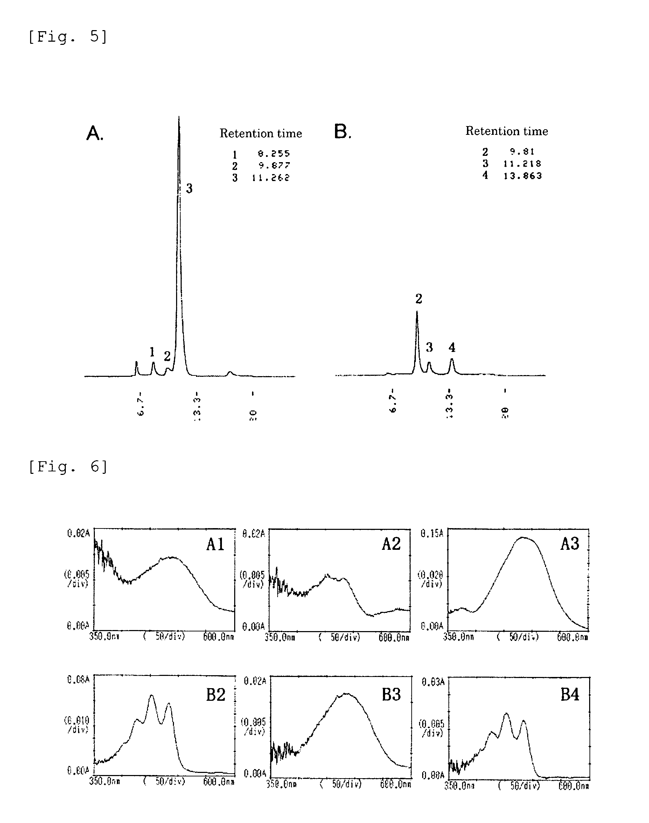

With regard to the objects where a carotenoid pigment composition is analyzed, *Rv. sulfidophilum* W-1S strain and pigment variant crtA-1 were selected. Pigments were extracted from them by the above-mentioned method and each pigment peak was detected via an HPLC analysis (Fig. 5). From the relation between main peaks and retention time, correspondence of carotenoid pigments such as spheroidenone (SO), spheroidene (SE) and derivatives thereof and neurosporene (NS) to each peaks was presumed [S.Takaichi, et al., (2001) Photosynth.Res., 67:207-14]. In addition, fractions corresponding to main peaks were prepared from discharged liquid passing through a detector and absorption spectra from 350 nm to 600 nm were measured (Fig. 6). As a result, the measured main peak showed characteristic absorption curve resembling to SE or SO [A.A.Yeliseev, et al., (1997) FEBS Letters, 403(1):10-4] and, when retention time was also taken into consideration, it was presumed that the peaks 1, 2, 3 and 4 would be SO derivative, SE derivative, SO and SE, respectively.

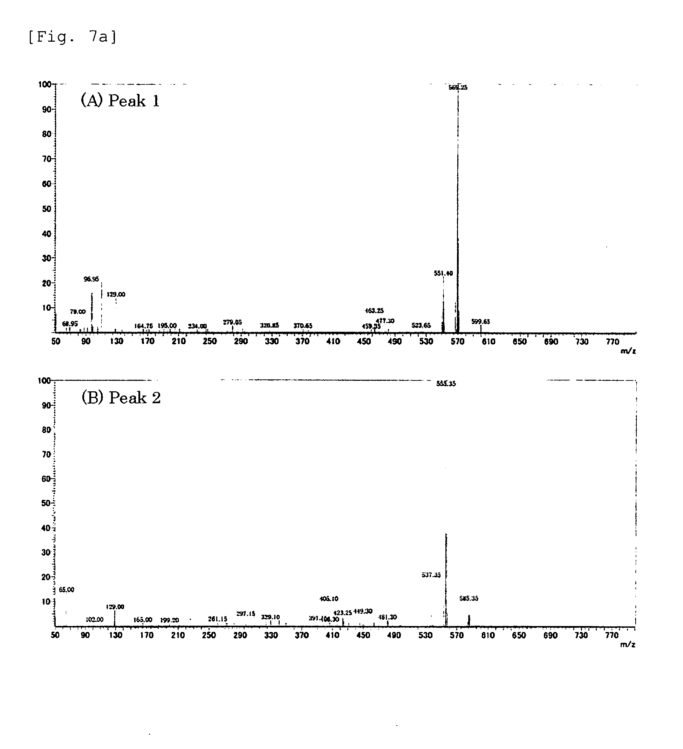

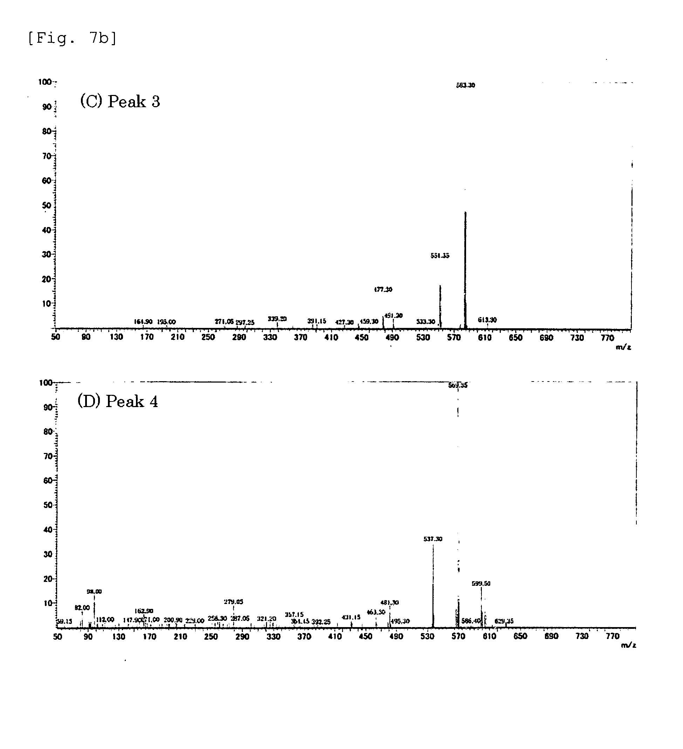

In order to confirm whether presumption for each of the peaks is correct and also to specify the substance corresponding to each carotenoid derivative, analysis of each main peak was carried out using a liquid chromatographic mass spectrometer (LC-MS). The result is shown in Fig. 7. In each of the peaks, m/z 569, 555, 583 and 569 showing the protonized molecules (M + H)⁺ were observed, respectively and molecular weights of those peak components were shown to be 568, 554, 582 and 568, respectively. Molecular weight of the peaks 3 and 4 coincided to SO and SE, respectively. In addition, from the molecular weights, the peaks 1 and 2 were thought to be demethylspheroidenone (DMSO) and demethylspheroidene (DMSE), respectively.

From the above results, it was suggested that, in *Rv. sulfidophilum* wild strain W-1S, accumulation was mainly noted in SO and then DMSO and then DMSE followed and, in a pigment variant crtA⁻1, DMSE was mainly accumulated and then SE and SO followed. The fact that SO was accumulated in the pigment variant crtA⁻1 was predicted by the fact that the culture liquid tended to red color as a result of a long-term incubation for several days. The above is likely to suggest that, in crtA⁻1 strain, CrtA activity remains although its extent was low. The possibility as such is also supported by the following fact.

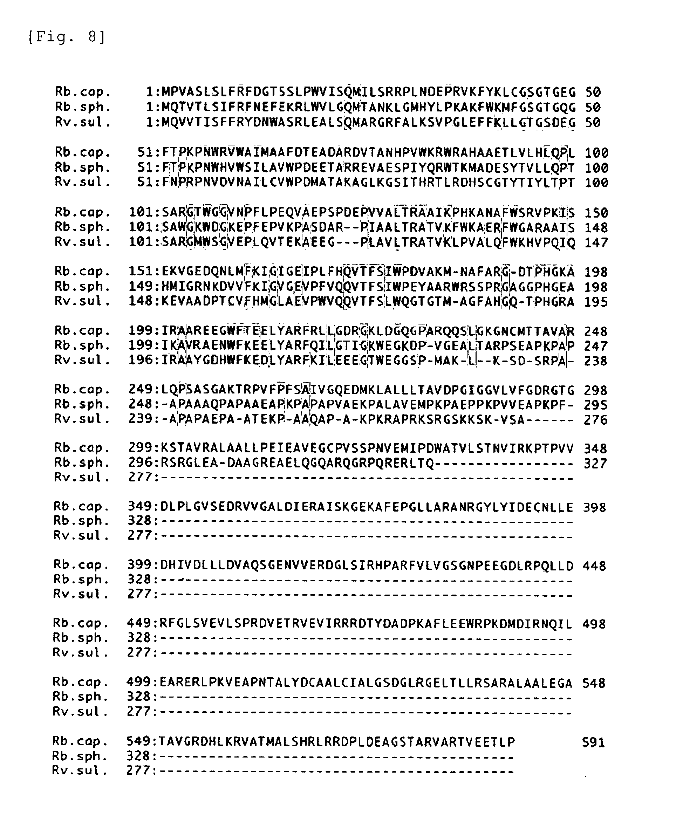

In preparing the pigment variant, a 1. 5-kb fragment containing crtA for which the present inventors succeeded in cloning was utilized. Km^{R} was inserted into a single restriction enzyme site located near the center of this fragment and a homologous recombination of this sequence with the crtA region on *Rv. sulfidophilum* genome was conducted whereupon the preparation of the pigment variant crtA⁻1 was in success. However, when a DNA sequence was subjected to a sequence decoding for the 1.5-kb fragment containing crtA, it was clarified that the fragment consisted of crtA gene region of about 830 bp as well as about 290 bp which was the upstream region thereof and about 400 bp which was the downstream region thereof and it was found that the XhoI site into which Km^{R} was inserted was present about 650 bp downstream from the crtA initiation codon. Thus, in a crtA::Km^{R} sequence constructed on the plasmid (or on the pigment variant genome), it was thought that, about 80% from the initiation codon, crtA was perfect. Therefore, in crtA⁻1 strain, it was presumed that, in CrtA sequence of the wild strain consisting of 277 amino acids in total, a 215 amino acid residue moiety from its N terminal was translated and it seemed that, due to the fact that a low spheroidene oxidizing activity derived by the partial CrtA was expressed, appearance of the peak corresponding to SO in an HPLC chart in crtA⁻1 was able to be explained. From the result of comparison of homology of CrtA amino acid sequences of *Rb*. *sphaeroides* and *Rhodobacter capsulatus* (GeneBank Accession No. AJ010302 and No. X52291, respectively) to a presumed amino acid sequence of CrtA of *Rv*. *sulfidophilum,* it was not possible to find a conserved region which was presumed to be an active center and it was unable to be said that CrtA deficiency was completely induced by insertion of Km^{R} (Fig. 8) . Accordingly, it is not possible to completely deny the aforementioned possibility that the partial CrtA of N terminal expressed in crtA⁻1 strain has an activity although its extent was weak.

As compared with the wild strain, CrtA activity in the pigment variant crtA⁻1 strain is greatly suppressed but, in a biosensor strain, it is likely that a low CrtA activity from the incompletely destroyed crtA on chromosome enhances the background upon measurement and greatly affects the measurement precision. Accordingly, it was thought to be necessary to construct a novel *Rv. sulfidophilum* pigment variant where crtA gene on genome was completely destroyed.

### 3. Summary

Result of this Example will be as follows.
. As a result of HPLC and LC-MS analysis, analysis of carotenoid pigment compositions of *Rv. sulfidophilum* wild strain and pigment variant was in success.
. As being presumed from the color tone, accumulation of SO was observed in crtA⁻1 strain and it was believed not to be appropriate to use this variant as a host for the biosensor.
From those results, in the construction of biosensor, it should be that a pigment variant where crtA gene on the genome is more completely deficient is constructed.

### Example 1-B Preparation of novel pigment variant

### 1. Object of the Example

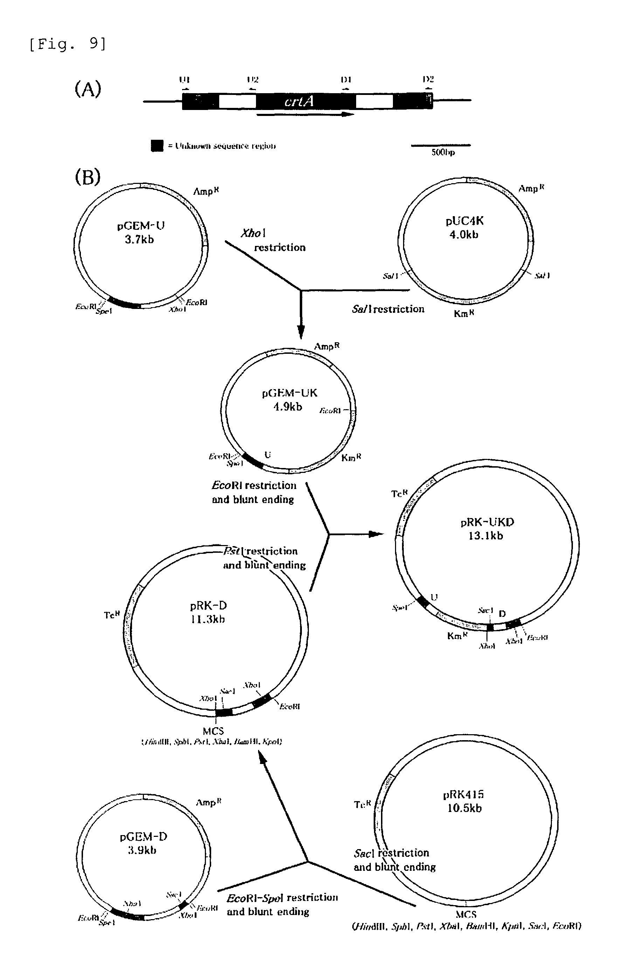

In the pigment variant crtA⁻1, construction of CrtA deficient strain was conducted in such a manner that Km^{R} was inserted into crtA gene on chromosome but the result was an incomplete CrtA deficiency causing accumulation of red pigment. Therefore, at this time, with an object of complete deficiency of CrtA, the region corresponding to crtA gene ORF was substituted with Km^{R} to construct a pigment variant (Fig. 9). The process was that, firstly, upstream region (U) and downstream region (D) of the crtA gene were cloned for the length of not less than 500 bp each. A fragment U-Km^{R}-D (fragment UKD) was constructed in such a manner that a Km^{R} sequence was inserted between those fragments and plasmid pHDR2 where the above was inserted on a suicide vector pSM3065 was prepared. The plasmid was introduced into *Rv. sulfidophilum* W-1S strain via conjugation to give the aimed pigment variant where homologous two-times recombinations took place.

Plasmids and primers which were used or constructed in this Example are summarized in Table 3 and Table 4, respectively.

**Table 3 Plasmids used in this Example**

| Plasmid | Characteristic |
|---|---|
| **PGEM-T Easy** | **Amp^{R};** Cloning vector |
| **PGEM-U** | **Amp^{R}; pGEM-T Easy** having the upstream region of ***crtA*** |
| **pGEM-D** | **Amp^{R}; pGEM-T Easy** having the downstream region of ***crtA*** |
| **pGEM-UK** | **Amp^{R}, Km^{R}; pGEM-U** having **Km^{R}** from **pUC4K** |
| **pGEM-UKD** | **Amp^{R}, Km^{R}; pGEM-T Easy** having Fragment **UKD** |
| **pUC4K** | **Amp^{R}, Km^{R};** Source of **Km^{R}** gene |
| **pRK415** | **Tc^{R};** Vector of broad host region |
| **pRK-D** | **Tc^{R}; pRK415** having Fragment **D** from **pGEM-D** |
| **pRK-UKD** | **Tc^{R}; pRK-D** having Fragment **UK** from **pGEM-UK** |
| **pSM3065** | **Tc^{R};** Suicide vector of **R6K** base |
| **pHDR2** | **Km^{R}, Tc^{R}; pSM3065** having Fragment **UKD** from **pRK-UKD** |

**Table 4 Sequences of primers used in this Example^{a}**

| Primer | Sequence | Restriction enzyme |
|---|---|---|
| **crtAdown1** | **5'-CTCGAGCAAGCTGAAATCGGACTCCA-3'** | ***Xho*I** |
| **crtAdown2** | **5'-GAGCACCATTTCTCCATGAAGGCGTC-3'** | |
| **crtAdown3** | **5'-GGACTAGTCATTTCTCCATGAAGGCGTC-3'** | ***Spe*I** |
| **crtAup 1** | **5'-CCGAGATCCTCGTAACCGAACCAGTA-3'** | |
| **crtAup2** | **5'-CTCGAGTTTGCGACTCTCCTAAACCG-3'** | ***Xho*I** |
| **crtAup3** | **5'-GGACTAGTTCCTCGTAACCGAACCAGTA-3** | ***Spe*I** |
| **kmR1** | **5'-GATAATGTCGGGCAATCAGGTG-3'** | |
| **kmR2** | **5'-AATCGCCCCATCATCCAGCCAGAA-3'** | |
| ^{a} Recognition site for restriction enzyme is in bolds. | | |

### 2. Preparation of novel pigment variant

For the cloning of both fragments U and D, primers crtAup1 and crtAup2 for amplification of Fragment U and primers crtAdown1 and crtAdown2 for amplification of Fragment D were designed. The primers crtAup2 and crtdown1 were designed on the basis of DNA sequence data of crtA while the primers crtAup1 and crtAdown2 were designed on the basis of DNA sequence data of *Rb*. *sphaeroides* on the database (Accession No.AJ010302). Although the crtA gene side of the fragment D contains a part of crtA gene (Fig. 9A) but, as same as the finding in *Rb*. *sphaeroides* [H.P.Lang, et al., (1995) J.Bacteriol., 177 (8) :2064-73], it has been also found from crtA gene sequence in *Rv. sulfidophilum* that stop codon of crtA gene and initiation codon of bchI (enzyme participating in the synthesis of bacteriochlorophyll) gene located at the downstream region thereof partially overlap and that, in the upstream region of bchI initiation codon, SD sequence is present overlapping with crtA gene coding region whereby, with an object of avoiding a damage to bchI, the part of crtA gene was not deleted but was made contained in the fragment D.

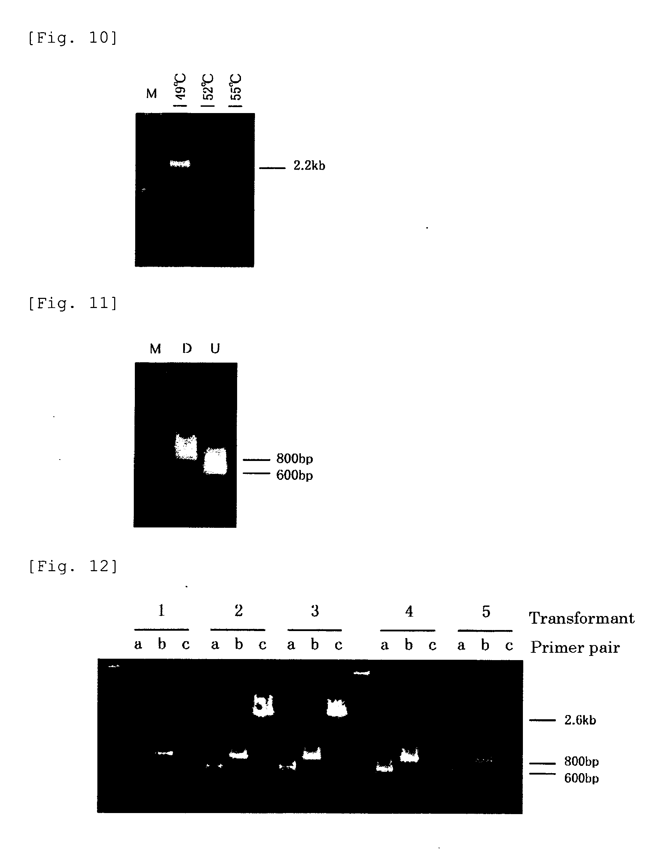

Firstly, a PCR was carried out using primers crtAup1 and crtAdown2 where *Rv. sulfidophilum* W-1S total DNA was used as a template (Fig. 10). It was confirmed by a PCR using a primer for crtA gene cloning that the crtA gene was contained in the resulting amplified fragment of about 2.2 kb. When a PCR was carried out using the primers crtAup1 and crtAup2 or the primers crtAdown1 and crtAdown2 where this amplified fragment of about 2.2 kb was used as a template, amplified fragment of 600 bp presumed to be a fragment U and amplified fragment of 800 bp presumed to be a fragment D were prepared, respectively (Fig. 11) . Each of the two fragments was inserted into pGEM-T Easy Vector and *E*. *coli* JM 109 strain was transformed. Sequence of the fragment inserted into the plasmid of the resulting transformant was decoded and compared with the known sequence of crtA gene, whereby it was confirmed that aimed fragment U and fragment D were cloned. Each of the resulting plasmids was called pGEM-U and pGEM-D, respectively. There will be two inserting directions for the cloning fragment into vector and a transformant keeping the aimed (Fig. 9) plasmid was selected by treating with a restriction enzyme by paying attention to XhoI site previously given to the primer crtAup2 as to pGEM-U and to SacI site in the already known sequence region of crtA_{Rv} as to pGEM-D.

After that, a fragment UKD was prepared on the broad host region plasmid pRK415 using cloned fragment U and fragment D and also Km^{R}.

Km^{R} was excised from the plasmid pUC4K by a treatment with a restriction enzyme SalI and inserted into XhoI site of pGEM-U plasmid. Insertion of Km^{R} into the plasmid was confirmed by the resistance of *E*. *coli* JM109 transformant to Km contained in the medium and by confirmation of the presence of Km^{R} by a PCR using primers kmR1 and kmR2. The constructed plasmid was called pGEM-UK.

Further, a fragment D was excised from pGEM-D plasmid by treating with SpeI-EcoRI, made into blunt end using a T4 DNA polymerase (New England Biolabs), treated with SacI and inserted into a pRK415 plasmid which was also made into blunt end. Insertion of the fragment D was confirmed by a blue-white judgment using X-Gal and by a PCR using primers crtAdown1 and crtAdown2. In the fragment D, there are two ways in its inserting directions into the plasmid and aimed one was selected by treating with a restriction enzyme EcoRI-SacI. The constructed plasmid was called pRK-D.

A fragment UK was excised from the plasmid pGEM-UK by treatment with EcoRI, made into blunt end and inserted into PstI site of the plasmid pRK-D which was also made into blunt end. With regard to the inserting direction of the fragment UK, there are also two ways. In the resulting plasmids, the aimed one produces amplified fragment corresponding to fragment UKD by PCR using the primers crtAup1 and crtAdown2 while amplified fragment is not confirmed in another one. A PCR was carried out to a plasmid of each transformant utilizing the above difference (Fig. 12) whereby aimed plasmid pRK-UKD was able to be obtained.

Fragment UKD was transferred from the resulting plasmid pRK-UKD to a plasmid pSM3065 and, in order to construct a plasmid pHDR2, fragment UKD (about 2.6 kb) was once amplified from the plasmid pRK-UKD using newly designed primers crtAup3 and crtAdown3 and a plasmid pGEM-UKD was constructed via a TA cloning. Fragment UKD which was excised by treating the plasmid pGEM-UKD extracted from the transformant with a restriction enzyme SpeI was mixed with pSM3065 fragment which was ring-opened by treating with XbaI and ligated. The ligation reaction solution was purified by means of extracting with PCI and precipitating with ethanol and then a microbe suspension of *E*. *coli* S17-1λ-pir was added thereto followed by subjecting to electroporation so that the plasmid was introduced. As a result, colonies were observed on an LB agar plate containing Tc and Km and, from the result of confirmation of the presence of fragment UKD by an antibiotic resistance mode and a PCR, it was believed that aimed plasmid pHDR2 was constructed.

After that, the plasmid was introduced into *Rv*. *sulfidophilum* W-1S via conjugation and, after a homologous recombination, a pigment variant was prepared.

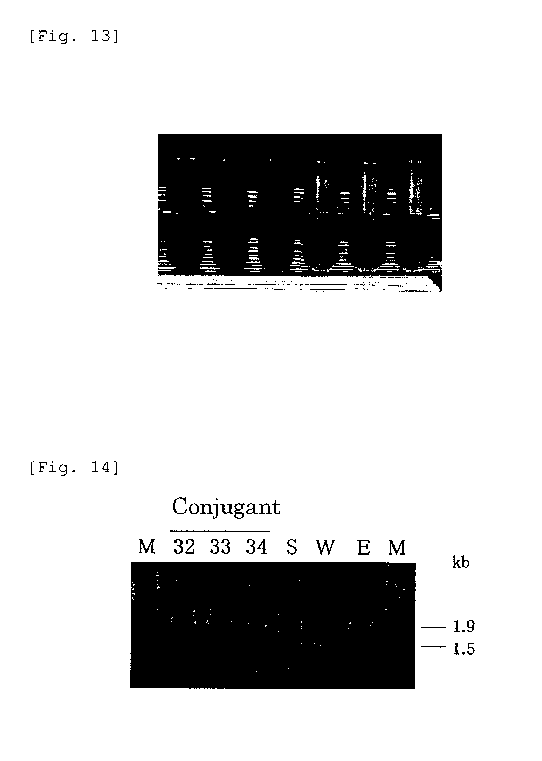

As a result of the conjugation, five kinds of colonies in reddish brown, orange, yellowish brown, yellow and white colors were observed on the plate. The colony in orange color turned reddish brown after some more incubation period. Further, when the colony in yellowish brown color was picked up and a liquid culture was continued whereupon it turned reddish brown. Those colonies in orange, yellowish brown and reddish brown colors were believed to be a single crossed strain judging from their color tone and from their Km resistance. The colony in white color is a recombinant *E*. *coli* used for the conjugation. The colony in yellow color did not become tinged with red even when picked up and a liquid culture was continued therefor and it will be highly probably an aimed double crossed pigment variant. The yellow colonies prepared at this time were three colonies and each of them was called crtA⁻32, crtA⁻33 and crtA-34 strains, respectively (Fig. 13).

crtA gene of each of those three pigment variants was confirmed by a PCR. The primer used therefor was that which was used for crtA gene cloning. It was presumed that, from crtA gene of a wild type, amplified fragment of 1. 5 kb will be observed and, from the sequence substituted with Km^{R} fragment, amplified fragment of 1.9 kb will be prepared. As a result where the PCR was actually conducted (Fig. 14), amplified fragment of 1.5 kb was observed in *Rv. sulfidophilum* W-1S while, in a recombinant *E. coli* used for the conjugation, amplified fragment of 1.9 kb of the region contained in fragment UKD was observed. From the yellowish brown clone which was presumed to be a single-cross pigment variant, two bands of 1.5-kb amplification and 1.9-kb amplification were confirmed. All of crtA⁻ 32 strain, crtA⁻ 33 strain and crtA⁻ 34 strain prepared at this time showed only 1.9-kb amplified fragment and were suggested to be the aimed double-cross strains.

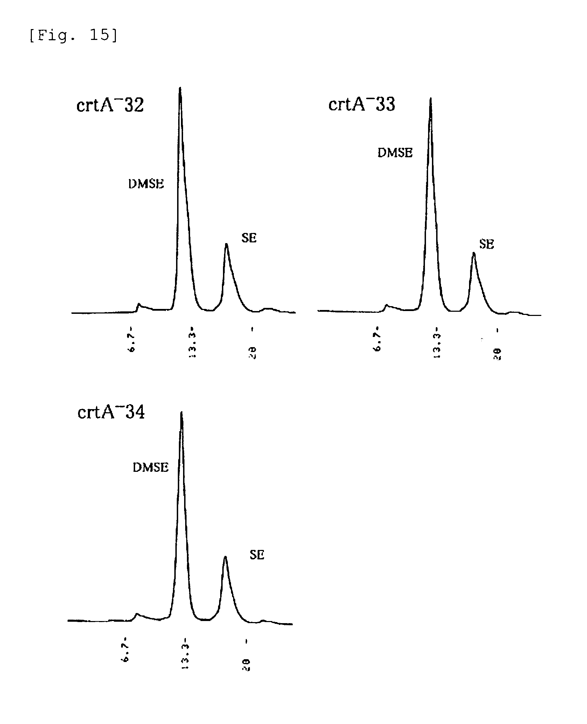

After that, carotenoid pigment compositions of those three strains were analyzed. Each of the three strains was cultured for one night and freeze-dried and pigment was extracted therefrom. Operation for the pigment extraction and condition for an HPLC analysis were the same as those which were conducted before. As a result of the HPLC (Fig. 15), no significant peak for SO was found for all of the three strains but high peaks for DMSE and SE were noted. It is judged that there is no problem in terms of function of pigment variant which is a basis for the biosensor system.

### 3. Summary

Result of this Example will be as follows.
. Fragment UKD was inserted on a suicide vector pSM3065 and the construction of plasmid pHDR2 was in success.
. When the plasmid pHDR2 was introduced into *Rv*. *sulfidophilum* W-1S strain, no reddish brown color of the wild strain was shown but a novel pigment variant showing yellow color was obtained.
. As a result of confirmation by PCR, in crtA gene region of this pigment variant, a band corresponding to the thing where ORF of crtA gene was partly substituted with Km^{R} was found and no band corresponding to a wild type crtA as noted in a single-cross pigment variant was found.
. As a result of pigment composition analysis using HPLC, no significant accumulation of SO was found in the resulting novel pigment variant.
From those results, the resulting novel pigment variants crtA⁻32, crtA⁻33 and crtA⁻34 are able to be said to be the aimed variants as a result where a homologous double recombination took place. From the pigment composition and stable color tone thereof, those pigment variants were believed to be applicable to biosensors.

### Example 1-C Construction of crtA vector

### 1. Object of the Example

As mentioned hereinabove already, three elements of host bacterium, reporter gene and inductive promoter sequence are essential in a biosensor. Among them, the fact that *Rv*. *sulfidophilum* pigment variant being a host bacterium for a biosensor system which is the object at this time was successfully constructed was summarized in the above Example. Now, in this Example, investigation of reporter gene and construction of crtA vector as the next steps for the construction of sensor system will be illustrated.

A sensor plasmid used for the construction of biosensor is constituted from broad host region vector, inductive promoter, reporter gene arranged at the downstream region of promoter and terminator hairpin sequence inserted for prevention of read-through derived from promoter other than inductive promoter to the reporter gene. It is an object of this Example to construct crtA vector which is a plasmid at the stage before the sensor plasmid and the stage where the inductive promoter is not inserted and to investigate its function. For this reporter gene, ORF of crtA is used while, for the terminator sequence, that on the database being shown to be of presence in gene sequence which has been already reported in *Rv. sulfidophilum* is used. Besides the aimed crtA vector, vector of a type having a promoter sequence of crtA is also constructed. That is to confirm whether CrtA deficiency of the pigment variant is complemented by crtA expressed from the plasmid.

Plasmids and primers used or constructed in this Example are summarized in Table 5 and Table 6, respectively.

**Table 5 Plasmids used in this Example**

| Plasmid | Characteristic |
|---|---|
| **pGEM-T Easy** | **Amp^{R};** Cloning vector |
| **pGV2** | **Amp^{R}; pGEM-T Easy** having ***crtA*** gene |
| **pRK415** | **Tc^{R};** Vector of broad host region |
| **pRKΩ** | **Tc^{R}; pRK415** having an improved terminator sequence |
| **pRV1Ω** | **Tc^{R}; pRKΩ** having ***crtA*** (consisting of ***P*_{crt}, SD** and **ORF**) |
| **pRV5Ω** | **Tc^{R}; pRKΩ** having ***crtA*** (consisting of **SD** and **ORF**) |

**Table 6 Sequences of primers used in this Example ^{a}**

| Primer | Sequence | Restriction enzyme |
|---|---|---|
| **crtArv1** | **5'-AACGTCGAGCTTGTCGATGA-3'** | |
| **crtArv2** | **5'-ttcctctagaCGGTTTAGGAGAGTCGCAAA-3'** | ***Xba* I** |
| **crtArv3** | **5'-aaggtaccAGAAGGGAAAGGGTTGAGTC-3'** | ***Kpn* I** |
| **term1** | **5'-ggaaagcttGCGCCGcGcGGGctcCtccCCgCgCGGCGC** | ***Hind* III** |
| | **TTTTTTTctgcagaa-3'** | ***Pst* I** |
| **term2** | **5'-TTCTGCAGAAAAAAAGCGCC-3'** | ***Pst* I** |

| | | |
|---|---|---|
| ^{a} Nucleotides in small letters show changes conducted in the sequence. Recognition site for restriction enzyme is in holds. | | |

### 2. Construction of crtA vector

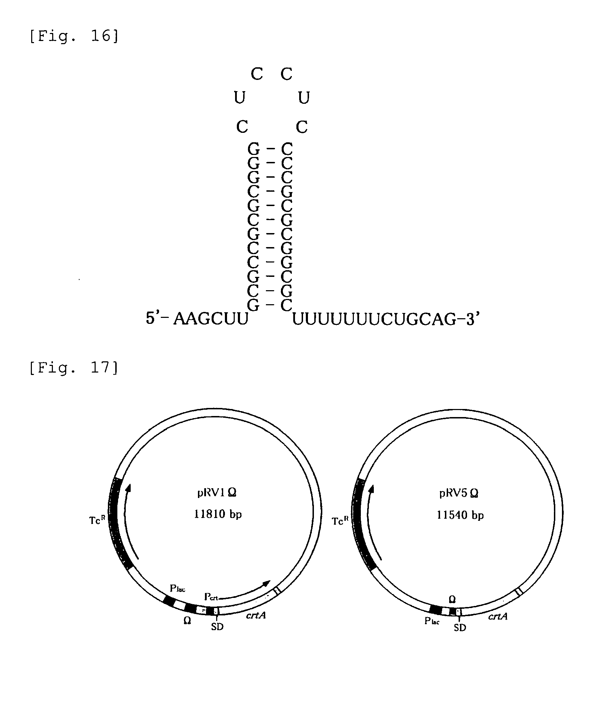

Construction of crtA vector was carried out in accordance with the following strategy.
The terminator sequence used was designed by referring to that which is present in the downstream region of pufC gene of puf operon on genome of *Rv. sulfidophilum* W-1S [L.Vasilyeva, et al., (1999) Appl.Biochem.Biotechnol., 77-79:337-45; S.Masuda, et al., (1999) J.Biol.Chem., 274(16):10795-801]. On the basis of hairpin structure existing in the downstream region of pufC gene, an oligonucleotide consisting of the sequence of 5'-GGAAAGCTTGCGCCGCGCGGGCTCCTCCCCGCGCGGCGCTTTTTTTCTGCAGAA-3' as a term1 sequence was designed (Fig. 16). In this, 100% of complementation is possible at the stem area of the hairpin and restriction enzyme sites (Hind III, PstI) are designed on both sides. In addition, with regard to an Ω::SD::crtA ORF sequence where the case of construction of crtA vector is presumed using this sequence, the fact whether a promoter-like sequence is able to be resulted is tested using GENETYX-WIN Version 4.0.0 (Software Development K. K.) program and, with regard to the area on the hairpin structure where similarity to promoter sequence is relatively high, design is conducted with an appropriate change in the base sequence. By using the above-prepared sequence as a template, an extension reaction of 5 cycles in a way of PCR using a primer term2 complementary to 3' end of the term1 was performed, whereupon an Ω sequence of 54 bp was prepared. After the fragment was purified, it was subjected to a treatment with a restriction enzyme HindIII-PstI for one night so that both ends were made into sticky ends. This fragment was inserted into HindIII-PstI site existing on the plasmid pRK415 to construct a pRKΩ plasmid. It was confirmed by decoding the DNA sequence. Into this plasmid was inserted a crtA fragment to construct a crtA vector.

After the crtA fragment was amplified by means of a PCR using *Rv. sulfidophilum* W-1S genome as a template, it was inserted onto pGEM-T Easy Vector via a TA cloning. In this PCR, the primers crtArv1 and crtArv3 were used. The fragment amplified by this PCR contained a sequence from about 300 bp upward of the crtA initiation codon to about 30 bp downward of the stop codon and that was confirmed by the fact that the pattern of the fragment obtained by the treatment of this fragment with a restriction enzyme was identical with that presumed from sequence data. Thus, crtA gene ORF, SD sequence and promoter (Pert) were contained in this crtA sequence. The plasmid constructed by insertion of this fragment was called pGV2.

Each of full-length and partial sequences of the crtA fragment on the plasmid pGV2 was purified and inserted into pRKΩ plasmid to construct two kinds of plasmids (Fig. 17). One of them was a pRV1Ω plasmid containing an Ω::P_{crt}::SD::crtA ORF sequence constructed by such a manner that a full-length crtA sequence on the pGV2 plasmid was excised by treating with a restriction enzyme SphI-SpeI, blunted at its ends by treating with a T4 DNA polymerase and ligated to an EcoRI site which was blunted similarly at its ends. It is likely that, when the plasmid pRV1Ω is introduced into a pigment variant, crtA gene is expressed from the plasmid and it is presumed that, as a result thereof, defect of CrtA in the pigment variant is compensated to result in a red transformant. Another was a pRV5Ω plasmid containing an Ω::SD::crtA ORF sequence constructed via the same process as above from a fragment using the primers crtArv2 and crtArv3 where the pGV2 plasmid was used as a template. This plasmid is an aimed crtA vector and, if no SO accumulation is noted in a pigment variant into which pRV5Ω is introduced, the outcome is that the Ω sequence suppresses a read-through derived from P_{lac} on the plasmid without no problem in terms of functions.

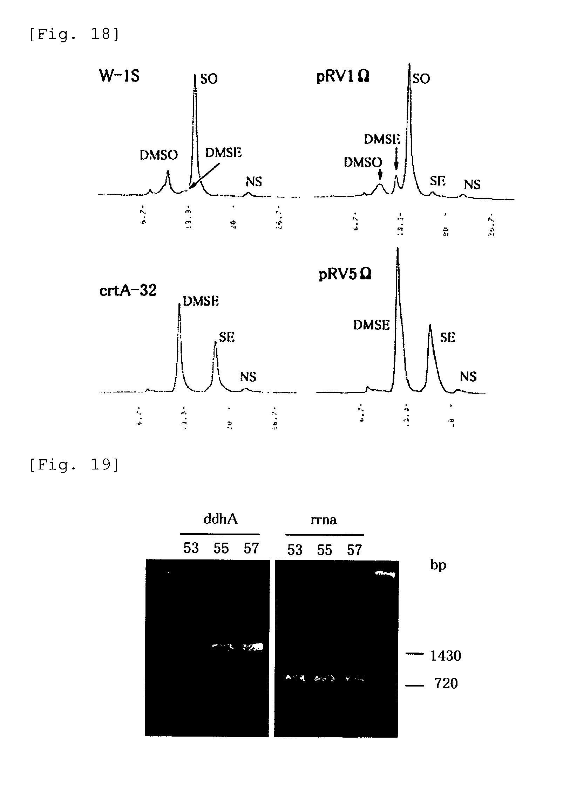

Each of those two kinds of plasmids was introduced into a pigment variant crtA⁻32 strain via conjugation. Colonies obtained on a 4CyMOM agar plate containing 30 µg/ml of Km and 5 µg/ml of Tc were isolated and incubated in a liquid medium containing the same concentrations of the antibiotics. The cells were recovered from the culture liquid and freeze-dried for one night and, after extraction of pigment therefrom, an HPLC analysis was carried out (Fig. 18). In a strain into which the plasmid pRV1Ω was introduced, a significant SO accumulation was observed and, as expected, it was likely that crtA expressed from the plasmid complemented the defect of CrtA in the pigment variant. From the above, it may be said that utilization of the crtA gene as a reporter gene is possible in a pigment variant. In a transformant where a pRV5Ω plasmid having an SD sequence was introduced into an upstream area of crtA gene ORF, accumulation of SO was rarely noted. Since an SD sequence is present, it will be presumed that the transformant expresses crtA when read-through from P_{lac} happens but SO accumulation was not observed and it will be concluded that the terminator sequence used at this time achieves the aimed function and is able to suppress the read-through derived from P_{lac} whereby construction of the aimed crtA vector was in success.

### 3. Summary

Result of this Example will be as follows.
. It was found to be possible to utilize crtA gene as a reporter system based on a change in color tone from yellow to red to a pigment variant due to CrtA defect.
. It was shown that a terminator sequence having excellent function was designed and the sequence was able to suppress the transcription from its upstream area.
. Construction of pRV5Ω plasmid into which crtA ORF sequence containing Ω sequence and SD were inserted was in success. It was confirmed that, from this plasmid, no crtA expression took place including a read-through.
From those results, it is able to be said that construction of crtA vector which is the second step in the process of construction of biosensor was in success. It is likely that a sensor plasmid is able to be constructed when an inductive promoter is inserted into the crtA vector.

### 5. Conclusions

Contents of Example 1 will be summarized to be as follows.
1. Construction of a CrtA defective pigment variant in such a form that most of crtA gene ORF on *Rv. sulfidophilum* W-1S genome were substituted with a kanamycin-resisting gene cassette was in success. No accumulation of red pigment in this pigment variant was found.
2. As a result of insertion of a plasmid pRV1Q having a crtA promoter on the upstream area of crtA gene into *Rv*. *sulfidophilum* pigment variant and analysis of pigment composition, it was found that, in the pigment variant, crtA gene of *Rv. sulfidophilum* was able to be utilized as a reporter gene.

### Example 2

### Example 2-A Sulfur compound-inductive promoter

### 1. Object of the Example

In *Rv. sulfidophilum,* there were few genes which have been reported up to now and clarified for their sequences and those in which participation of transcription regulation for their expression has been made clear are limited. Among the information limited as such, there is information concerning a DMS dehydrogenase of *Rv. sulfidophilum.*

DMS (dimethyl sulfide) is an odor substance which is utilized as a starting material for various organosulfur compounds such as pharmaceuticals and agricultural chemicals or as an odor-giving agent. In the scale of global environment, it is contained in a sulfur cycle and has been known to be produced in the ocean by phytoplankton and oxidized after being discharged into the air to give sulfuric acid. Due to its low vapor pressure, sulfuric acid becomes particles and forms condensation nuclei for clouds. The fact that sunlight is interrupted and reflected by the cloud formation has been receiving public attention from the viewpoint of suppression of global warming and, with a purpose of a search for contribution to weather variation of the earth, investigation of changes in DMS concentration in the ocean has been carried out already [M.Horinouchi, et al., (1999) Nippon Nogeikagaku Kaishi, 73(1):35-44].

When the above is taken into consideration, there will be detection of pollution by DMS in a few amount, test whether removal of DMS was properly conducted, search of changes in DMS on a global environment level, etc. as the uses for which DMS sensor is applied.

In this Example, investigation whether a promoter of a DMS dehydrogenase gene reported in *Rv. sulfidophilum* is suitable for utilization to biosensors is carried out, the promoter is cloned if good data are found and construction of a sensor plasmid is aimed.

Plasmids and primers which were used or constructed in this Example are summarized in Table 7 and Table 8, respectively. Other experimental materials and methods used in this Example are the same as those in Example 1.

**Table 7 Plasmids used in this Example**

| Plasmid | Characteristic |
|---|---|
| **pGEM-T Easy** | **Amp^{R}; Cloning vector** |
| **pGEM-I34** | **Amp^{R}; pGEM-T Easy** having an inverse **PCR** product |

**Table 8 Sequences of primers used in this Example ^{a}**

| Primer | Sequence | Restriction enzyme |
|---|---|---|
| **ddhA1** | **5'-TCGATCCATTCCGATCTGTG-3'** | |
| **ddhA2** | **5'-ATCGACGAAGAAGGTGATCC-3'** | |
| **rrna1** | **5'-AGCCTACGATCTATAGCTGG-3'** | |
| **rrna2** | **5'-GGAATTCCTCTCGGAAAACC-3'** | |
| **crtAup 1** | **5'-CCGAGATCCTCGTAACCGAACCAGTA-3'** | |
| **crtArv3** | **5'-AAGGTACCAGAAGGGAAAGGGTTGAGTC-3'** | ***Kpn* I** |
| **i-ddhA1** | **5'-TGTCGCCGATGGGTTCAAGC-3'** | |
| **i-ddhA2** | **5'-TCTGCATGGCGACGCTGTTG-3'** | |

| | | |
|---|---|---|
| ^{a} Recognition site for restriction enzyme is in bolds. | | |

### 2. DMS dehydrogenase gene operon

DMS dehydrogenase is an enzyme carrying the reaction where DMS is oxidized to give dimethyl sulfoxide and, up to now, its presence has been confirmed in *Rv. sulfidophilum* and an enzyme protein has been purified [S.P.Hanlon, et al., (1994) Microbiol., 140:1953-8; S.P.Hanlon, et al., (1996) Eur.J.Biochem., 239:391-6]. It has been suggested that the DMS dehydrogenase is an enzyme belonging to the same family as a dimethyl sulfoxide reductase carrying the reaction where dimethyl sulfoxide is reduced to DMS and it has been proved that expression of enzyme protein is induced by the presence of a substrate. Thus, it has been confirmed that induction of DMS dehydrogenase of *Rv*. *sulfidophilum* by DMS is able to take place on a level of protein expression. In recent years, a gene operon (ddhABDC) coding for a DMS dehydrogenase protein has been identified [C.A.McDevitt, et al., (2002) Mol.icrobiol., 44 (6) :1575-87] and its base sequence is also able to be utilized from databases (Accession number AF453479). In an upstream area of ddh operon, presence of a ddhS gene sequence which is thought to code for DdhS presumed to be similar in terms of N-terminal amino acid sequences to DorS protein which is one of a binary control system (DorR and DorS) of a dimethyl sulfoxide reductase system of *Rb*. *sphaeroides* has been also suggested. However, information concerning ddh operon registered in the databases is limited to the downstream area from ddhA gene initiation codon and no information has been laid-open for its promoter sequence and for more upstream area thereof. Accordingly, when a promoter (P_{ddh}) of a ddh operon is applied to a biosensor, the following two points are necessary. They are (i) P_{ddh} induces the transcription of downstream gene in the presence of DMS and (ii) cloning of upstream area of ddhA is conducted to make its sequence clear.

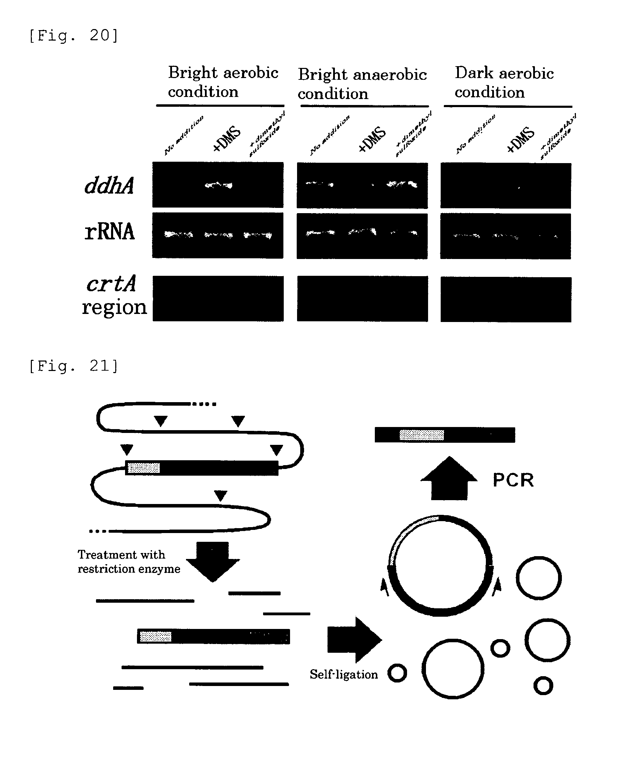

Firstly, investigation of a DMS-inductive property of P_{ddh} was planned. Primers ddhA1 and ddhA2 were designed based on ddhA gene sequence on the database. In addition, primers rrna1 and rrna2 were designed based on an rRNA sequence information of *Rv. sulfidophilum* W-1S (Accession number U55277). It was confirmed that a PCR using those primers was able to amplify about 1.5-kb region contained in the ddhA gene region or about 700 bp of rRNA sequence (Fig. 19). The fact that those amplified fragments were the aimed fragments was confirmed from the pattern of fragment prepared by the treatment with each of plural kinds of restriction enzymes. Those primers were used for analysis of transcription induction of ddhA gene by addition of DMS via an RT-PCR.

*Rv. sulfidophilum* W-1S was incubated for 3 days in a 4CMOM medium under the condition where DMS was added, dimethyl sulfoxide was added or neither of them was added thereto and the cells were recovered from the culture liquid during a logarithmic growth phase. RNA was extracted therefrom, treated with DNase, purified and subjected to an RT-PCR. When ddhA and rRNA were used as a target in the PCR, cycles were set at 40 cycles and 20 cycles, respectively. In order to confirm that no genomic DNA was mixed therein, an RT-PCR using primers crtAup1 and crtArv3 was carried out as well. The target of this RT-PCR is the region from 600 bp upstream of crtA gene initiation codon to downstream area of stop codon of crtA ORF and it is likely that transcription of this region is not done throughout the total length. As a result of the RT-PCR (Fig. 20), an apparent ddhA gene transcription induction was observed by addition of DMS under a bright aerobic condition and a dark aerobic condition. In that case, no mixing-in of genomic DNA was confirmed. In addition, no significant induction of ddhA gene transcription by addition of dimethyl sulfoxide was observed although such a tendency was noted only under a bright anaerobic condition. Under the condition where no sulfur compound was added, a low (although not zero) ddhA transcription activity was noted in any of a bright aerobic condition and a dark aerobic condition and it is likely that suppression that P_{ddh} is receiving is not very strong. Further, under a bright anaerobic condition, a relatively strong ddhA-inductive activity was noted even when no sulfur compound was added and, therefore, a possibility that not only by control with DMS but also by intracellular oxidation-reduction equilibrium participates in P_{ddh} was suggested. That is similar to the presence of a route regulated via optical stimulation and Redox controlling system and an activating route by dimethyl sulfoxide suggested in a binary control system participating in dimethyl sulfoxide reductase gene regulation of *Rb. sphaeroides* [N.J.Mouncey, et al., (1998) J.Bacteriol., 180(21):5612-8].

From the above, a significant transcription induction of ddhA gene under a bright aerobic condition by addition of DMS was confirmed and it is likely that, when P_{ddh} is used, construction of DMS biosensor is possible.

### 3. Cloning of promoter P_{ddh}

After that, P_{ddh} which will be present in the upstream area of ddhA was cloned and its base sequence was decoded. The region is a region where its sequence has been unknown being adjacent to a ddhA gene where its sequence has been known already and, therefore, an inverse PCR [H.Ochman, et al., (1988) Genetics, 120: 621-3] which is a means suitable for cloning of such a region was used. This means will be briefly mentioned as hereunder (Fig. 21). In this means which is excellent in cloning both or one sequence(s) adjacent to the gene region where the sequence has been known already, treatment of genomic DNA with a single restriction enzyme is the first step. The resulting fragment as such was subjected to a self-ligation to cyclize and, using it as a template, a PCR was conducted using a primer which was designed outwardly seen from the conventional gene orientation based on the gene sequence where the sequence has been known already. The fragment prepared by the amplification is a sequence which specifically contains the region where the sequence is unknown and it is easy to clone it.

ORF of ddhA gene has been reported to be 2,733 bp. Target region of the primers ddhA1 and ddhA2 corresponds to a region of from +801 to +2240 when the ddhA initiation codon is defined +1 and, in the downstream area thereof, the only SalI site in ddhA ORF is present at the position of +2713. Therefore, when *Rv. sulfidophilum* W-1S strain genome was subjected to a SalI treatment, most of unknown sequences of upstream side of ddhA and ddhA ORF are contained in a fragment which is in a size of at least 2,700 bp and detection of the fragment will be possible using the primers ddhA1 and ddhA2. Based on that, it was decided to clone the upstream area of ddhA gene via the aforementioned inverse PCR means.

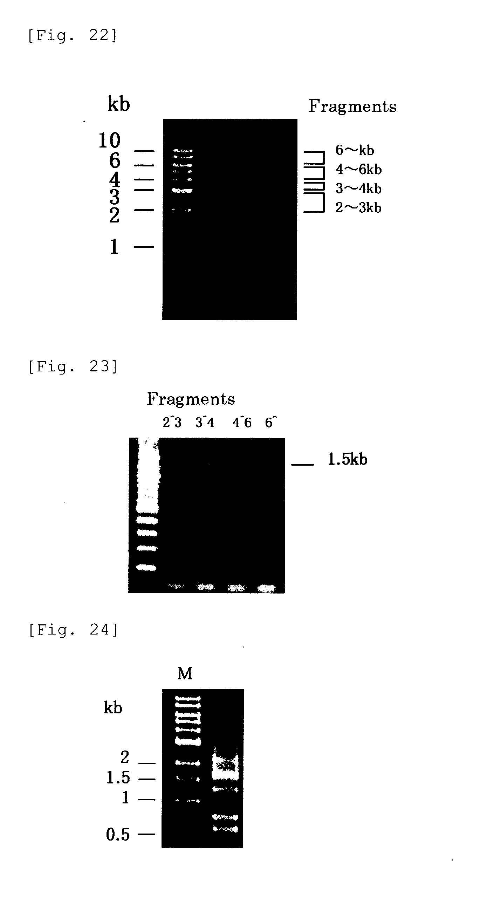

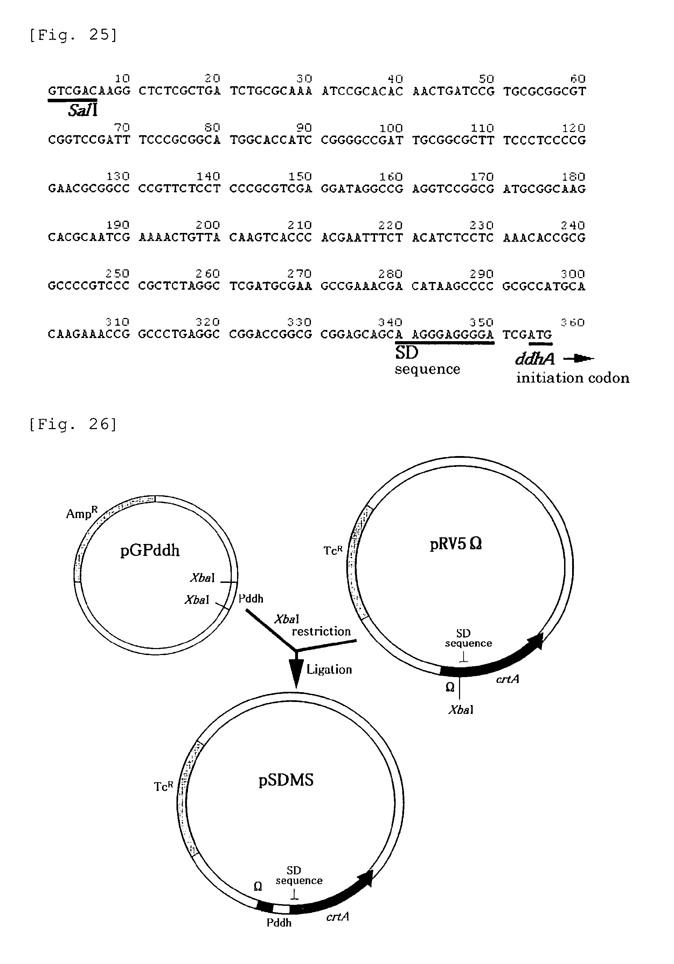

At first, genomic DNA of *Rv. sulfidophilum* W-1S was treated with SalI for one night, the reaction solution was subjected to a 1% agarose gel electrophoresis and four fractions of 2~3 kb fraction, 3~4 kb fraction, 4~6 kb fraction and 6 kb and higher fraction were excised therefrom (Fig. 22). PCR was then conducted using the primers ddhA1 and ddhA2 by the use of a mixed-in DNA fragment contained in each fraction as a template (Fig. 23). As a result, it was confirmed that ddhA gene was present at least in the 3-4 kb fraction. A ligation reaction was carried out for one night to this 3-4 kb fraction DNA solution. An inverse PCR was carried out using the primers i-ddhA1 and i-ddhA2 where the above reaction solution was used as a template (Fig. 24). A TA cloning of the fraction which was now succeeded in the amplification as such was tried whereupon cloning of about 600 bp fragment was successful and the plasmid was called pGEM-134. Sequencing was conducted using i-ddhA1 and i-ddhA2 as primers and, as a result, it was made clear that the fragment inserted into the pGEM-134 plasmid was constituted from 453 bp of upstream area containing an upstream area of ORF of ddhA gene and 109 bp of downstream area of ORF of ddhA gene. Fig. 25 shows a sequence (SEQ ID No. 1) of a ddhA upstream area from SalI site to ddhA initiation codon which was made clear as a result of the sequencing. On a pGEM-134 plasmid, ddhA gene ORF at the upstream area from the position of +2713 was ligated at the upstream area from this SalI site.

From the analysis of about 350 bp region which was succeeded in cloning, an SD sequence of ddhA was found and, as a result of analysis using a GENETYX-WIN program, two things which were presumed to be promoter sequences were found near -150. However, it was not possible to find a motif suggesting the system participating in the control of activity of promoter and a sequence similar to Dor Box [N.J.Mouncey, et al., (1998) J.Bacteriol., 180 (11) :2924-30] which is a bonding region of a binary control system on genome found in dimethyl sulfoxide reductase operon of *Rb*. *sphaeroides.* Although it was not possible to specify the controlling region, there will be a relatively high possibility that P_{ddh} is contained in this region when the promoter-like region found in the fragment which was cloned at this time and a report for upstream area of ddhA which was previously conducted [C.A.McDevitt, et al., (2002) Mol.icrobiol., 44 (6) : 1575-87 are taken into consideration and it was planned to construct a sensor plasmid in which this region was used as a P_{ddh} region.

### 4. Summary

Result of this Example will be as follows.
. It was found that transcription of downstream gene by a promoter P_{ddh} of ddh gene operon coding for a DMS dehydrogenase was induced in the presence of DMS.
. Cloning of about 350 bp in upstream area of ddhA gene was in success and its base sequence was made clear.
. Although it was still ambiguous for a control system, a region which is thought to be P_{ddh} was able to be found.
It is presumed that an aimed P_{ddh} is contained in the upstream area of ddhA whose cloning was in success at this time and it is likely that, when this region is inserted into a crtA vector, construction of a sensor plasmid which is necessary for construction of a DMS biosensor is possible.

### Example 2-B Construction of DMS biosensor

### 1. Object of the Example

Up to now, preparation of pigment variant, construction of crtA vector containing reporter gene and cloning of DMS-inductive promoter have been carried out as constituting elements of a biosensor. In this Example, construction of a biosensor was carried out as the final stage and its function was evaluated.

Aiming for the construction of biosensor, an inductive promoter is firstly incorporated into a crtA vector to construct a sensor plasmid and then the sensor plasmid is introduced into a pigment variant. The pigment variant prepared as such is likely to have a function which is predicted as a biosensor or, in other words, no crtA expression takes place under the condition where no DMS is added but the color tone is the same as the pigment variant and, when DMS is added, color tone of the culture liquid changes by crtA expression.

The plasmids and the primers which were used or constructed in this Example will be shown in Table 9 and Table 10, respectively.

**Table 9 Plasmids used in this Example**

| Plasmid | Characteristic |
|---|---|
| **pGEM-T Easy** | **Amp^{R};** Cloning vector |
| **pGEM-I34** | **Amp^{R}; pGEM-T Easy** having an inverse **PCR** product |
| **pGPddh** | **Amp^{R}; PGEM-T Easy** having an upstream area of ***ddhA*** |
| **pRK415** | **Tc^{R};** Vector of broad host region |
| **pRV5Q** | **Tc^{R}; pRK415** having terminator and ***crtA*** (consisting of **SD** and **ORF**) |
| **pSDMS** | **Tc^{R}; pRK5Ω** having ***Xba*I** fragment from **pGPddh** |

**Table 10 Sequences of primers used in this Example^{a}**

| Primer | Sequence | Restriction enzyme |
|---|---|---|
| **crtArv3** | **5'-AAGGTACCAGAAGGGAAAGGGTTGAGTC-3** | ***Kpn* I** |
| **pddh1** | **5'-TCTAGAAAGGCTCTCGCTGATCTGCG-3'** | ***Xba* I** |
| **pddh2** | **5'-TCTAGACCGGTCCGGCCTCAGGGCCG-3'** | ***Xba* I** |

| | | |
|---|---|---|
| ^{a} Recognition site for restriction enzyme is in bolds. | | |

### 2. Preparation of DMS biosensor strain

PCR was carried out using the primers pddh1 an pddh2 where a plasmid pGEM-134 was used as a template. This targets about 320 bp from SalI site up to upstream area of SD sequence of ddhA among the upstream area of ddhA on the template plasmid. No SD sequence is contained in this sequence. It was confirmed that the amplified fragment was an aimed sequence by comparing the pattern of the fragment produced by a restriction enzymatic treatment with the sequence data of upstream area of ddhA. This fragment was subjected to a TA cloning onto a pGEM-T Easy Vector to construct a plasmid pGPddh. This fragment was excised by an XbaI treatment and inserted into an XbaI site existing between Ω of a plasmid pRV5Ω which is a crtA vector and crtA ORF to construct a sensor plasmid pSDMS (Fig. 26) . With regard to insertion of a P_{ddh} fragment into the constructed plasmid, two patterns will be available in view of orientation of the sequence thereof and that where amplified fragment is produced by a combination of the primer pddh1 with crtArv3 and no amplified fragment is produced by a combination of primer pddh2 with crtArv3 when PCR was conducted extracting DNA from the transformed colony was decided to be a clone retaining the aimed plasmid.

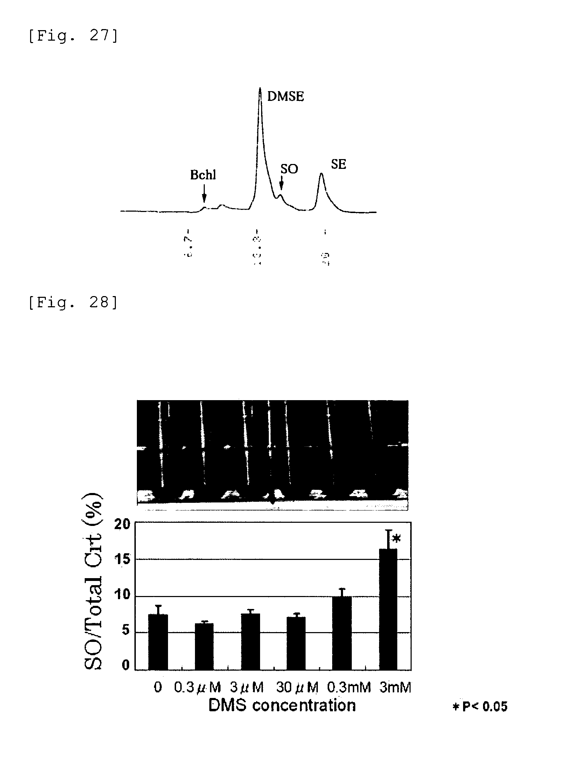

After that, the sensor plasmid which was in success for its construction was introduced into a pigment variant via conjugation. The resulting transformant formed yellowish brown colonies. From the fact of showing resistance to Km and Tc and from the PCR, it was able to be confirmed that a sensor plasmid was introduced into the transformant. This transformant was incubated for 3 days under a bright aerobic condition without addition of DMS to analyze the pigment composition. As a result thereof (Fig. 27), an HPLC chart similar to the pigment composition of the pigment variant as a whole was obtained. Accumulation of SO was also observed although only a little and that is able to be explained by a weak ddhA transcription activity when no sulfur compound was added which was observed in Fig. 18 of the previous Example. So far as a DMS sensor is concerned, there will be no problem if induction upon addition of DMS is in such a significant level that it greatly exceeds the above-mentioned slight accumulation of SO.

3. Evaluation of functions of DMS biosensor strain After that, DMS was added to the constructed biosensor to evaluate its functions. Firstly, DMS was added in the initial stage of the incubation and it was checked whether the response to DMS was available.

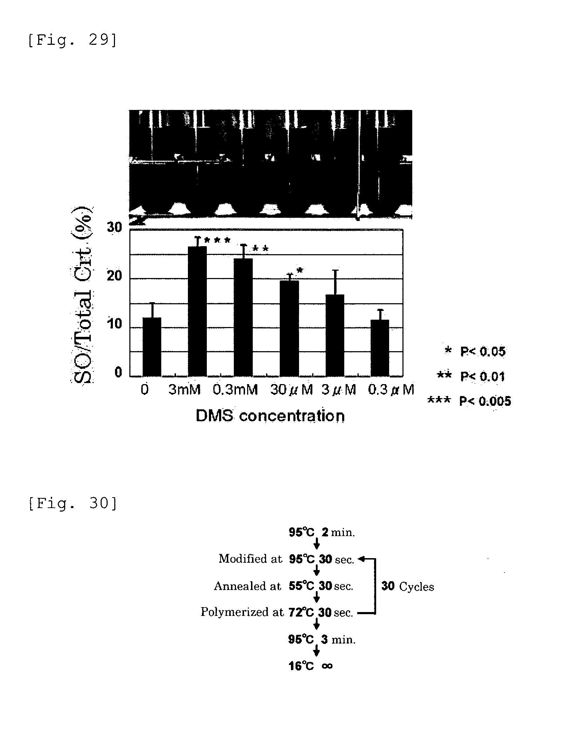

Since DMS is hardly soluble in water, ethanol was used as a solvent for the manufacture of concentrated stocks and preparation of diluted series. DMS-containing ethanol was added to the culture liquid in an amount of 0.1% by volume and, with regard to a control, only ethanol was added in an amount of 0.1% by volume. In each sample, final concentration of the DMS added was made 3 mM, 0.3 mM, 30 µM, 3 µM or 0.3 µM and, in all samples, experiment was conducted where n = 3. The incubation was carried out under a bright aerobic condition and, on the third day (logarithmic growth phase) and on the sixth day (resting phase), observation of color tone and pigment composition analysis from collected cell sample were carried out. Analysis of pigment composition was conducted via an HPLC analysis the same as before and, from the value of area of each peak shown in the resulting HPLC chart, rate of red pigments (DMSO and SO) to the sum of total carotenoid pigment (DMSO, DMSE, SO, SE and neurosporene) was calculated. For each sample, mean value and standard deviation was calculated from the above value and a graph was prepared. Color tone and rate of red pigment on the third day of the incubation are shown in Fig. 28 and those on the sixth day are shown in Fig. 29. On the third day of the incubation, a significant accumulation of SO was observed especially in the sample to which 3 mM DMS was added and its color tone was also able to be discriminated as compared with the control. From the above, it is suggested that the promoter sequence used at this time contains the aimed P_{ddh} and it is now apparent that the constructed biosensor strain has a function as "visual biosensor" which is the initial object. On the sixth day of the incubation, a significant SO accumulation was observed in a sample where DMS concentration was 30 µM (2 ppm) and concentration-dependency in DMS response and response in very little amount are suggested.

### 4. Summary

Result of this Example will be as follows.
. It was found that a ddh operon promoter P_{ddh} was contained in an upstream sequence of ddhA gene.
. Construction of a DMS sensor plasmid was in success.
. Since a pigment variant into which a DMS sensor plasmid was introduced showed a significant red pigment accumulation in the incubation under the condition where DMS was added, it is likely that construction of a DMS biosensor was in success.
. Changes in color tone by a DMS response of a DMS biosensor strain reached a level being recognizable by naked eye and construction of "visual biosensor system" was in success.

### 5. Conclusions

Contents of Example 2 will be summarized to be as follows.
1. It was made clear that transcription of a ddh gene operon coding for a DMS dehydrogenase was induced by the presence of DMS and cloning of upstream area of ddhA containing the promoter sequence was in success.
2. Construction of "visual biosensor" accompanied by color tone change from yellow to yellowish brown responding to DMS was in success by the joint use of pigment variant, crtA gene and ddhA promoter.

### Example 3

### Materials and methods for experiments

### 1: Strains and media used in this Example

Strains used in this Example are shown in Table 11 while media used therein are shown in Table 12.
A 4CyMOM medium was used for incubation of *Rv*. *sulfidophilum* W-1S strain and variants and transformant strains thereof. For the incubation of *E*. *coli* JM109 strain, E. *coli* S17-1 strain and transformant strain for each of them, an LB medium was used. If necessary, ampicillin (Amp), kanamycin (Km) or tetracycline (Tc) was added to each medium so as to make the final concentration thereof 50 µg/ml, 30 µg/ml or 20 µg/ml, respectively. With regard to a *Rv. sulfidophilum* however, the amount of Tc to be added was adjusted so as to make its final concentration 5 µg/ml.
An SOC medium was used during a temporary incubation during the transformation process.

**Table 11 Strains used in this Example**

| Strain | Origin or reference documents |
|---|---|
| ***Rhodovulum sulfidophilum* W-1S** | 2 |
| ***Rhodovulum sulfidophilum* crtA**⁻32 | 5 |
| ***Eschelichia coli* JM109** | **TaKaRa** |
| ***Escherichia coli* S17-1** | 3 |
| ***Escherichia coli*** K-12 MG1655 | ATCC 47076 |

**Table 12 Composition of media used in this Example**

| | | | |
|---|---|---|---|
| LB Medium | | **4CyMOM** | |
| Tryptone peptone | **10g** | **NaCl** | **30g** |
| Yeast extract | **5g** | **CaCl₂ · 2H₂O** | **0.2g** |
| **NaCl** | **10g** | **MgSO₄ · 7H₂O** | **0.25g** |
| Distilled water was added to make the total amount 1 liter | | **FeSO₄ · 7H₂O** | **0.02g** |
| | | **KH₂PO₄** | **40.8mg** |
| SOC Medium | | **K2HP04** | **495mg** |
| | | Thiamine | **0.5mg** |
| SOB medium | **1L** | Nicotinic acid | **0.5mg** |
| **1M MgCl₂** | **10ml** | **PABA** | **0.3mg** |
| **1M MgSO₄** | **10ml** | Biotin | **0.05mg** |
| **1M** Glucose | **20ml** | Sodium succinate | **1.0g** |
| SOB Medium | | Sodium DL-malate | **1.0g** |
| | | Sodium acetate | **1.0g** |
| Tryptone peptone | **20g** | Sodium pyruvate | **1.0g** |
| Yeast extract | **5g** | Yeast extract | **1.0g** |
| **NaCl** | **0.5g** | **1M NH₄Cl** | **5.0ml** |
| **250mM KCl** | **10ml** | **A'5** solution | **1.0ml** |
| **5N NaOH** | **0.2ml** | Distilled water was added to make the total amount 1 liter | |
| Distilled water was added to make the total amount 1 liter | | Adjusted to pH 8.0 | |
| | | **A'5** solution | |
| | | **H₃BO₄** | **2.86g** |
| | | **MnCl₂ · 4H₂O** | **1.81g** |
| | | **ZnSO₄ · 7H₂O** | **0.22g** |
| | | **CuSO₄ · 5H₂O** | **0.08g** |
| | | **Na₂MoO₄** | **0.021g** |
| | | **CoCl₂ · 6H₂O** | **0.01g** |
| | | **EDTA · 2Na** | **50g** |
| | | Distilled water was added to make the total amount 1 liter | |

### 2: Extraction of total DNA

Extraction of total DNA from *Rv. sulfidophilum* W-1S strain was carried out according to the protocol of the reference document ["Illustrated Bio-Experiments, (ii) Fundamental Gene Analysis" (1995) by Takahito Nishikata, published by Shujunsha].

### 3: PCR

Operations of a PCR were carried out using TaKaRa LA Taq^{™} with GC Buffer (manufactured by TaKaRa) in accordance with a protocol attached thereto. With regard to a template, each total DNA or plasmid DNA was used or a culture liquid or a solution in which colonies were suspended was used. In the latter case, 50 µl of culture liquid or a solution where colonies were suspended in 50 µl of H₂O was heated at 95°C for 5 minutes and a direct PCT was carried out using a supernatant liquid prepared by centrifugal separation as a template. A fundamental program of the PCR is shown in Fig. 30. In this program, annealing temperature and time for extension reaction were appropriately modified in each PCR.

### 4: Recovery of DNA fragment from gel, ligation and transformation

When the fragment amplified by a PCR is cloned, it was incorporated into pGEM-T Easy Vector and then introduced into *E. coli* JM109. The method was carried out in accordance with the protocol for pGEM-T Easy Vector System. In case a plasmid DNA was introduced into *E*. *coli* S17-1, the method therefor also followed said protocol.
The DNA fragment treated with a restriction enzyme was subjected to electrophoresis on agarose gel and then recovered by an excision from the gel. In extracting the DNA from the gel fragment, that was conducted using a dialysis tube ["A Laboratory Manual - Genetic Engineering" edited by Masahiro Muramatsu, (1988), published by Maruzen]. The liquid in the dialysis tube was recovered by paying careful attention so that gel did not come therein and the DNA was recovered by extracting with PCI (phenol-chloroform-isoamyl alcohol) ["Illustrated Bio-Experiments, (i) Fundamental Molecular Biochemical Experiments" (1995) by Takahito Nishikawa, published by Shujunsha] and by precipitating with ethanol. In the operation of precipitating with ethanol, glycogen (Nakarai Tesk) was used as a co-precipitating agent for the purpose of enhancing the recovering efficiency of DNA.
When the excised DNA fragment is subjected to ligation, it was conducted using DNA Ligation Kit Ver. 2.1 (TaKaRa).

### 5: Extraction of plasmid DNA

Extraction of plasmid DNA was carried out using CONCERT Rapid Plasmid Miniprep System (GIBCO BRL), QIAprep Spin Miniprep Kit (QIAGEN) or QIAGEN Plasmid Midi Kit (QIAGEN).

### 6: Conjugation: Conjugation was carried out upon introduction of the plasmid DNA into Rv. sulfidophilum from Escherichia coli into which a recombinant plasmid was introduced. The method therefor is as shown below. Incidentally, all of the things used in the following operation were subjected to sterilization using an autoclave.

*E. coli* was subjected to a shake culture at 37°C in 10 ml of an LB medium in a test tube while *Rv. sulfidophilum* was incubated under a bright condition by stirring with a stirrer in 30 ml of 4CyMOM medium in a Roux bottle. *E. coli* was incubated until OD₆₆₀ became nearly 0.2 and *Rv. sulfidophilum* was incubated until OD₆₆₀ became nearly 1.6 and then 170 µl and 60 µl, respectively, of each microbe liquid was mixed in an Eppendorf tube. After collecting the cells, they were suspended again in 230 µl of an LB medium and the resulting liquid was spread on an MF-Millipore Type HA Membrane Filter (Millipore) on 2% NaCl LB agar plate. After the plate was allowed to stand overnight at 32°C, the membrane was recovered into a vial. 4CyMOM (250 µl) was added into the vial and subjected to vortex whereupon the cells on the membrane were collected, washed and suspended again in 4CyMOM. The cell liquid was applied on 4Cy MOM agar plate and incubated for several days under a bright condition.

### 7: DNA sequence

For decoding of a base sequence of the fragment cloned on the pGEM-T Easy Vector, there were used Fluorescein-Labeled Primer RV-M and M4 (TaKaRa) and ABI PRISM BigDye Terminator Cycle Sequencing Ready Reaction Kit With AmpliTaq DNA Polymerase, FS (Applied Biosystems) and Sequence ABI PRISM 310 Genetic Analyzer (Applied Biosystems). Preparation of the sample was carried out according to a protocol attached to the kit while operation of the sequencer was carried out according to the manual attached to the instrument.

### 8: Pigment analysis

Composition analysis of various carotenoid pigments of wild strain, variant strain and transformant strain of *Rv*. *sulfidophilum* was carried out by the following method.
The bacteria strain was incubated for one night together with stirring by a magnetic stirrer under slightly aerobic and bright condition in a Roux bottle in which 30 ml of medium was placed. Then, 1 ml of microbe liquid was collected, and after a centrifugal separation of 14,000 rpm for 5 minutes using portable centrifuge HITACHI CENTRIFUGE SCT15B (HITACHI KOKI), the supernatant liquid was discarded, cell pellets were frozen at -80°C for 30 minutes and the cells were freeze-dried for one night. The resulting freeze-dried product was transferred from a centrifugal tube to a vial, 150 µl of a 7:2 (v/v) mixture of acetone and methanol was added thereto and the mixture was subjected to vortex for two minutes [S.Takaichi, et al., (2001) Photosynth.Res., 67:207-14]. The suspension was filtered and used as a sample for a high-performance liquid chromatography (HPLC).
When incubation of a bacterium strain was conducted in 10 ml of medium in a test tube under various conditions (under slightly aerobic and bright condition; under slightly aerobic and dark condition; and under anaerobic and bright condition), the incubation was done for three days. Then, the culture liquid (1 ml) was placed in an Eppendorf tube, the pellets after centrifugal separation was frozen at -80°C for 2 minutes and then freeze-dried for one night. 150 µl of a mixed liquid of acetone and methanol was added to the resulting freeze-dried product, subjected to vortex for 2 minutes and then centrifuged for 2 minutes at 14,000 rpm using a portable centrifuge and the resulting supernatant liquid was used as a sample for an HPLC.
The HPLC analysis was carried out using instruments for high-performance liquid chromatography manufactured by Shimadzu, liquid-sending unit LC-10AS, autoinjector SIL-9A, detector for spectrophotometer for ultraviolet and visible regions SPD-10A and chromatopack C-R3A (Shimadzu) and µ Bondapack^{™} C18 125Å 10 µm 3.9 × 300 mm HPLC column (Waters). Methanol was used for a liquid phase and the detection was conducted with a measuring wavelength of 481 nm. Infusing amount of the sample, flow rate of the liquid phase, recording condition for chromatopack, etc. were appropriately modified depending upon the conditions such as sample concentration.

### 9: RNA extraction and RT-PCR

Extraction of RNA was carried out using RNeasy Mini Kit (QIAGEN) according to the protocol attached thereto. For dissolving the cells at that time, lysozyme was added thereto in the indicated concentration. For the removal of the contaminated DNA, DNase I (TaKaRa) was used by the method indicated in the protocol together with the buffer attached thereto. Incubation was carried out at 37°C for 60 minutes and, after that, RNA was purified via a PCI treatment and a precipitating operation with ethanol. A reverse transcription reaction was carried out where 1 µg of purified RNA as a template using an RNA PCR Kit (AMV) Ver. 2.1 (TaKaRa). In the reverse transcription reaction, things contained in the kit, i.e. reverse transcriptase, RNase inhibitor, random nonamer, buffer, dNTP and MgCl₂ were used and the reaction was conducted at 45°C for 28 minutes. A PCR was carried out by the same manner as in the above mentioned "3: PCR" using 4 µl of the solution after the reverse transcription reaction as a template.

### 10: Preparation of probe and southern hybridization

Preparation of probe was carried out using an AlkPhos Direct Labelling and Detection System (Amersham) according to the manual attached thereto.
Preparation of membrane used for the southern hybridization was carried out as follows. Firstly, 10 µg of genomic DNA was used and subjected to a treatment with each restriction enzyme for 24 hours. After that, it was subjected to electrophoresis at 40 V for 14 hours using agarose gel and then stained with ethidium bromide to confirm the bands. Then blotting was conducted to a Hybond N⁺ (Amersham) membrane by an alkali transfer method, washed with 2 x SSC and incubated at 80°C for 2 hours to fix. The fixed membrane was subjected to hybridization with a probe for 24 hours and, after washing, a fluorescent substrate was added thereto. Measurement of fluorescence was conducted using a FluorImager 595 (Amersham) .

### Example 3-A Selection of suitable arsenic-inductive promoter

### 1. Object of the Example

In order to prepare a biosensor for the detection of arsenic with high sensitivity, type of the arsenic-inductive promoter used therefor is important. With regard to that, it has been known that, particularly in bacteria, arsR which codes for control protein showing a response to arsenic in a very low concentration participates in the control of the expression of arsenic-resisting gene group ars operon [A.Carkin, et al., (1995) J.Bacteriol., 177 (4) :981-6] and, when it is utilized as a control region of the biosensor of the present invention, it is likely that a biosensor for the detection of arsenic in high sensitivity is able to be prepared.

Accordingly, in this Example, arsR showing a response to arsenic and being necessary for expression control of crtA will be tried to investigate from *Rv*. *sulfidophilum.*

The plasmids and the primers constructed in this Example are shown in Table 13 and Table 14, respectively.

**Table 13 Plasmids used in this Example**

| Plasmid | Characteristic |
|---|---|
| **pGEM-T Easy** | **Amp^{R};** Cloning vector |
| **pGPars** | **Amp^{R}; pGEM-T Easy** having **P*ars*** |
| **pRV1Ω** | **Tet^{R}; pRK415** having terminator and ***crtA*** (consisting of ***P*_{crt}**, **SD** and **ORF**) |
| **pRV5 Ω** | **Tet^{R}; pRK415** having terminator and ***crt*A** (consisting of **SD** and **ORF**) |
| **pRV5 Ω Pars** | **Tet^{R}; pRV5 Ω** having ***Xba*I** fragment from **pGPars** |

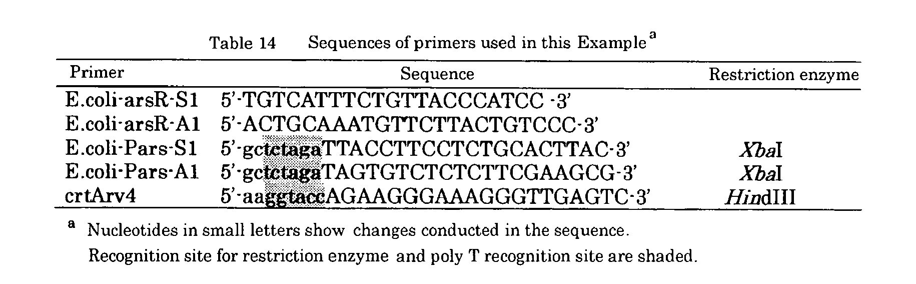

### 2. Investigation of arsR by southern hybridization

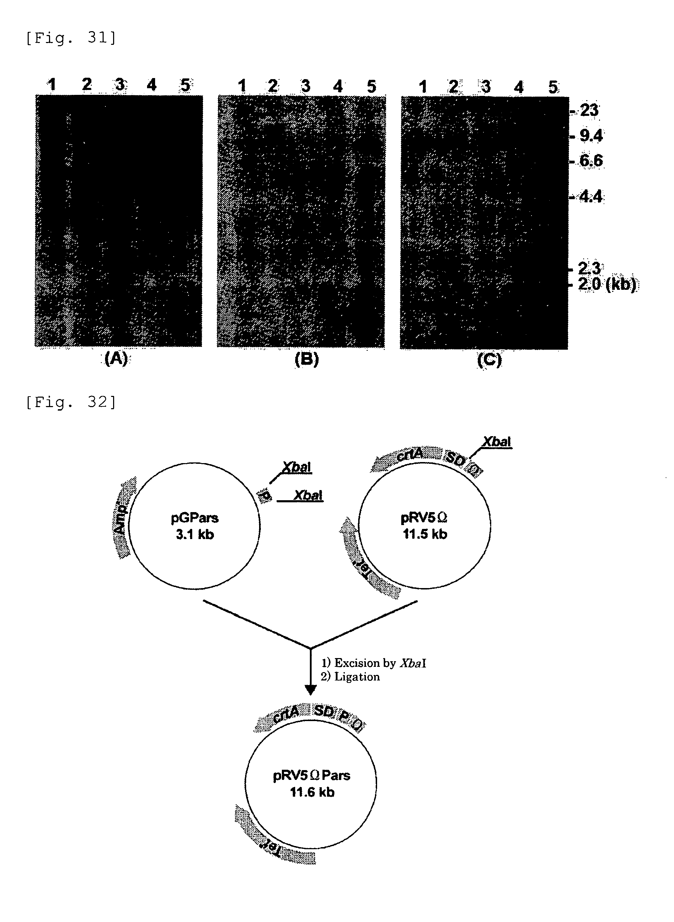

Firstly, it was confirmed whether arsenic-resisting gene was present in photosynthetic bacteria including *Rv*. *sulfidophilum* by conducting a blast search analysis using the base sequence information of *E*. *coli* arsR. As a result, it was found that *Rb*. *sphaeroides* which is a closely-related species to *Rv. sulfidophilum* had a sequence having a homology to ars RBC with a relatively high score. From the result, it was able to be presumed that *Rv. sulfidophilum* will also have an arsenic-resisting gene including arsR. Therefore, cloning of arsR of *Rv. sulfidophilum* was attempted by a southern hybridization where *E*. *coli* arsR was used as a probe. Firstly, a PCR was carried out using primers *E*. *coli*-arsR-S1 and *E*. *coli*-arsR-A1 where genomic DNA of E. *coli* K-12 was used as a template. The fragments obtained as a result thereof were purified, bonded with an alkaline phosphatase by a cross-linking reaction and used as a probe which was able to be detected by fluorescence. Hybridization of the probes was conducted at each of the temperatures of 50°C, 55°C and 60°C. As a result, although non-specific band was able to be detected, no signal believed to be arsR was unable to be obtained when hybridization temperature was 50°C. When hybridization temperature was 55°C and 60°C, no signal in the strength of positive control was able to be obtained in any of both cases. From the above, it was suggested that *Rv. sulfidophilum* had no sequence showing a homology to *E*. *coli* arsR (Fig. 31).

### 3. Evaluation of promoter activity of E. coli Pars in Rv. sulfidophilum

Taking the result of the above experiment into consideration, it is believed to be preferred for the construction of arsenic biosensors to use Pars and arsR of E. *coli* in which functions and sequences have been fully made clear.

Control of expression by arsR is inhibition of transcription by the bond of ArsR protein to the ArsR-bonding site contained in Pars. This bond is due to a physical interaction between DNA and protein and, even in the case where microorganism species in which Pars and arsR are expressed are different, it is believed that the same bonding property is available. A problem here is whether Pars derived from *E*. *coli* is recognized by an RNA polymerase of *Rv. sulfidophilum* whereby initiation of transcription takes place. In order to confirm that, it was decided to construct crtA vector in which Pars derived from E. *coli* was incorporated (Fig. 32) and to introduce it into a pigment variant via conjugation so as to confirm the function of the promoter. At this time, pRV1Ω where promoter Pcrt of *Rv. sulfidophilum* was incorporated was used as a positive control while, as a negative control, pRV5Ω where no promoter sequence was incorporated was used to evaluate the function of Pars.

Firstly, a PCR was carried out using primers *E*. *coli*-Pars-S1 and *E*. *coli*-Pars-A1 in which genomic DNA of *E*. *coli* K-12 was used as a template. After that, the fragments were subjected to a TA cloning on pGEM-T Easy Vector to construct a plasmid pGPars. The fragments were excised by an XbaI treatment and inserted into an XbaI site existing between Ω of a plasmid pRV5Ω which is a crtA vector and crtA ORF to construct a sensor plasmid pRV5ΩPars (Fig. 32) . For insertion of Pars fragment into the constructed plasmid, there will be two patterns depending upon the orientation of the sequence and that which produces amplified fragment by conducting a PCR using a primer *E*. *coli*-Pars-S1 and a primer crtArv4 for DNA extracted from the transformed colony was decided to be a clone retaining the aimed plasmid. Finally, a base sequence of 5' -upstream area of crtA of pRV5ΩPars was confirmed by sequencing.

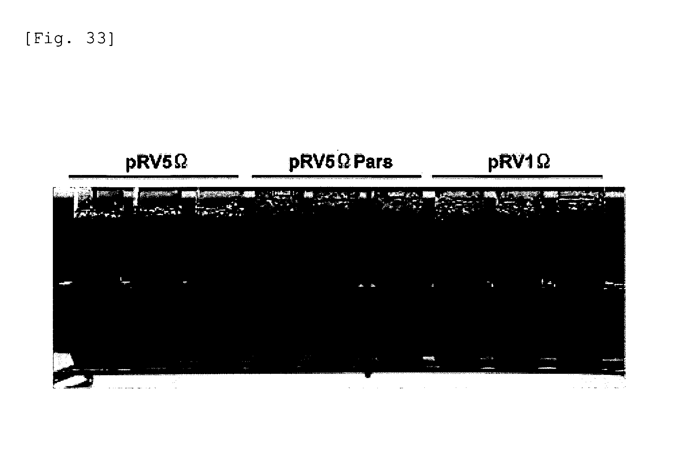

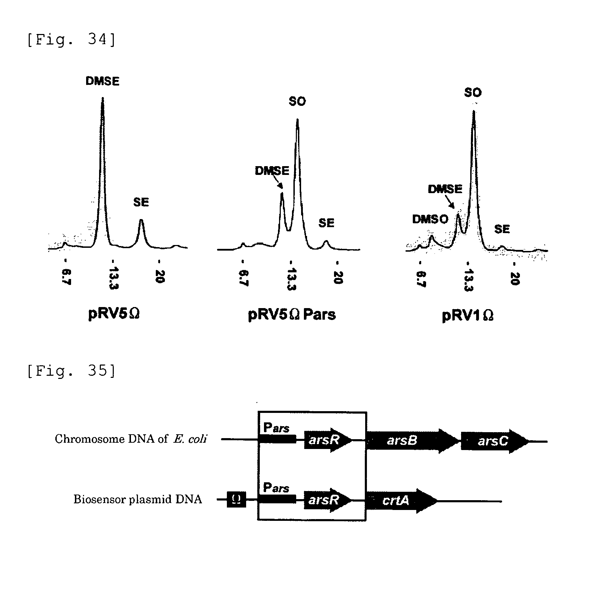

Then each of plasmids pRV5ΩPars, pRV1Ω and pRV5Ω which were successfully constructed was introduced into a pigment variant via conjugation and incubated for 3 days under a bright aerobic condition to give colonies. Each of the colonies was picked up and subjected to liquid culture for 3 days under a bright aerobic condition and the culture liquid was subjected to observation for its color tone and to analysis of the pigment composition by an HPLC. As a result, the culture liquid was in red color in the pigment variant into which the plasmid pRV5ΩPars was introduced and it is able to be expected the accumulation of SO took place by incorporation of Pars (Fig. 33). From the result of pigment analysis by an HPLC, in a pigment variant into which pRV5Q was introduced, accumulation of SO in the similar degree as in the case of a pigment variant into which no plasmid was introduced was able to be confirmed while, in a pigment variant into which pRV5ΩPars was introduced, accumulation of SO in the similar degree as in the case of pRV1Ω was able to be confirmed (Fig. 34). From those, it was suggested that E. *coli* Pars in *Rv. sulfidophilum* had the similar promoter activity to Pcrt.

### 4. Summary

Result of this Example will be as follows.
. It was suggested that no arsR was present in *Rv*. *sulfidophilum.*
. It was made clear that the promoter Pars of ars operon of E. *coli* was recognized by an RNA polymerase of *Rv*. *sulfidophilum* and functioned as a promoter.
From the above results, it was expected that, when Pars and arsR of *E*. *coli* were used as arsenic-inductive promoters, it was possible to construct an arsenic-detecting biosensor.

### Example 3-B Construction of arsenic-detecting biosensor

### 1. Object of the Example

From the results of Example 3-A, it was expected that, in the construction of arsenic-detecting biosensors, use of Pars and arsR of E. *coli* as arsenic-inductive promoters is effective. Accordingly, in this Example, construction of sensor plasmids using them was attempted. That which was constructed in this Example is a sensor plasmid where a sequence from Pars to immediately before the initiation codon of arsB (The sequence includes sequences of Pars and arsR and corresponds to the sequence of SEQ ID No. 29. Incidentally, the bases from the first to the 97th ones in SEQ ID No. 29 correspond to Pars and those from the 98th to the 451st ones therein correspond to arsR) was incorporated into upstream area of crtA and that was constructed with such an object that the same control as ars operon of a wild type is conducted in crtA as well (Fig. 35). It was also investigated whether a strain into which such a sensor plasmid was introduced functions as a biosensor.

The plasmids and the primers constructed in this Example are shown in Table 15 and Table 16, respectively.

**Table 15 Plasmids used in this Example**

| Plasmid | Characteristic |
|---|---|
| **pGEM-T Easy** | **Amp^{R};** Cloning vector |
| **pGASK** | **Amp^{R}; pGEM-T Easy** having **P*****ars*** and ***arsR* ORF** |
| **pRV5 Q** | **Tet^{R}; pRK415** having terminator and ***crtA*** (consisting of **SD** and **ORF**) |
| **pSENS-AS** | **Tet^{R}; pRV5Q** having ***Xba*I** fragment from **pGASK** |

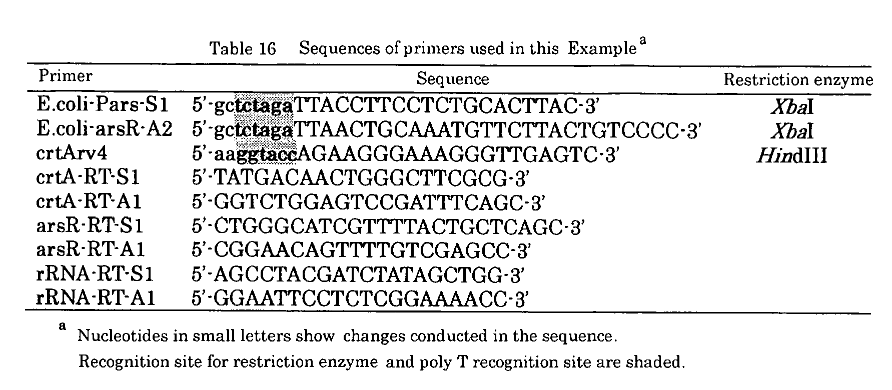

### 2. Construction of arsenic-detecting biosensor plasmid

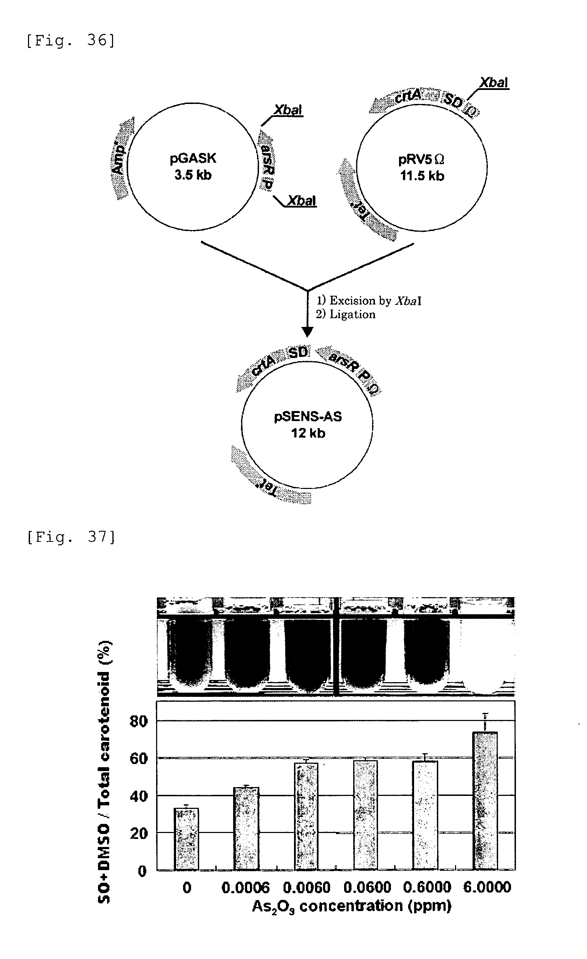

Firstly, a PCR was carried out by a combination of a primer *E*. *coli*-Pars*-S1* and a primer E. coli-arsR-A2 using genomic DNA of *E*. *coli* K-12 as a template. The resulting amplified sequence corresponds to a sequence (SEQ ID No. 29) from Pars to immediately before initiation codon of arsB. The fragment was subjected to a TA cloning onto pGEM-T easy vector to construct a plasmid pGASK. The fragment was excised by an XbaI treatment and inserted into an XbaI site existing between Ω of plasmid pRV5Ω which is a crtA vector and crtA ORF to construct a sensor plasmid pSENS-AS (Fig. 36). In that case, there will be two ways in a direction for insertion into the crtA vector and, therefore, confirmation of the direction by a PCR using a primer *E*. *coli*-Pars-S1 and a primer crtArv4 and confirmation by sequencing were carried out.

After that, the sensor plasmid which was in success for its construction was introduced into a pigment variant by conjugation to give an arsenic-detecting biosensor SENS-AS strain. Confirmation of introduction of the sensor plasmid was conducted by the fact of showing a resistance of Km to Tet and from the result of the PCR.

### 3. Evaluation of function of arsenic-detecting biosensor

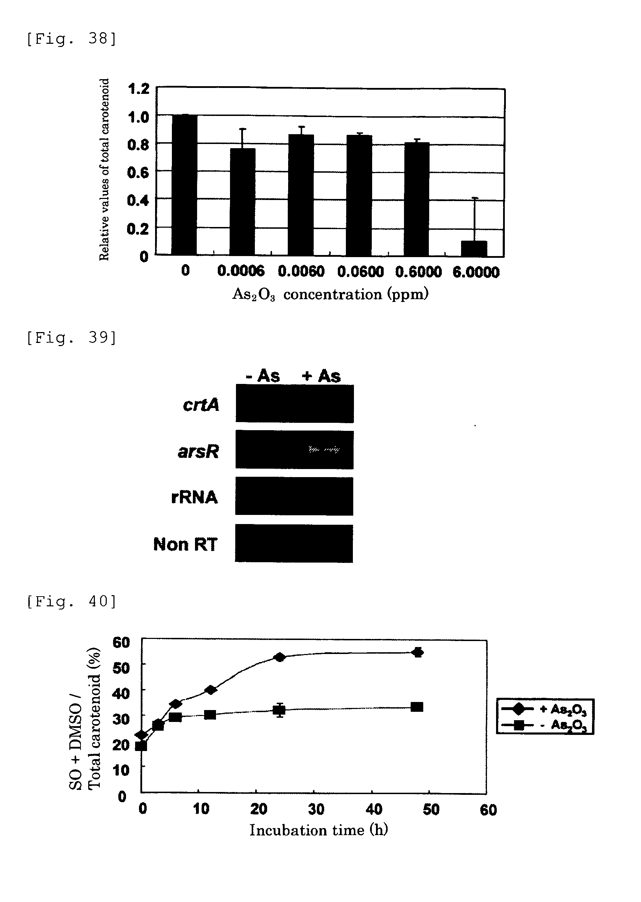

After that, arsenic was added to the constructed biosensor strain and the responding property was evaluated. At that time, arsenious acid (hereinafter, referred to as As₂O₃) was used as a trivalent arsenic. Addition of As₂O₃ was carried out so as to make the final concentration 0.0006 ppm, 0.0060 ppm, 0.0600 ppm, 0.6000 ppm or 6.0000 ppm and, in all samples, the experiment was conducted where n = 3. After 24 hours from the start of the incubation, an observation of color tone and a pigment composition analysis from the collected cell sample were conducted. Analysis of the pigment composition was conducted via an HPLC and rate of red pigments (DMSO and SO) to the sum of DMSO, DMSE, SO and SE was calculated from an area value of each peak shown in the resulting HPLC chart. Further, mean value and standard deviation were calculated for each sample and graphs were prepared. As a result, color tone changed from yellow to red when AS₂O₃ was added to the SENS-AS strain and the color tone change as such was able to be confirmed by addition of As₂O₃ within a concentration range from 0.0060 ppm to 0.6000 ppm (Fig. 37). From the result of analysis of the pigment composition by the HPLC, promotion of accumulation of red pigment by addition of As₂O₃ was able to be confirmed in all samples. However, when 6.0000 ppm of As₂O₃ was added, changes in color tone were unable to be confirmed. When the amount of carotenoid pigment contained in 1 ml of culture liquid was confirmed, a significant decrease was able to be confirmed only when 6.0000 ppm of AS₂O₃ was added (Fig. 38). The cause therefor will be that, as a result of inhibition of growth of SENS-AS strain itself due to toxicity of As₂O₃, biosynthesized amount of the carotenoid decreased whereby changes in color tone were unable to be confirmed.

Further, an RNA extraction and an RT-PCR were carried out for a sample of after 24 hours from addition of 0.06 ppm of As₂O₃ and changes in transcribed amount of mRNA to As₂O₃ were checked whereby the responding property was evaluated. PCR where a sample after the RT reaction was used as a template was carried out for crtA using a primer crtA-RT-S1 and a primer crtA-RT-A1; for arsR using a primer arsR-RT-S1 and a primer arsR-RT-A1; and for rRNA using a primer rRNA-RT-S1 and a primer rRNA-RT-A1. From the result thereof, it was able to be confirmed that transcription induction of crtA took place by addition of As₂O₃ and it was able to be presumed that expression control which was just the same as in the construct of a biosensor plasmid took place (Fig. 39). Even when no As₂O₃ was added, a weak transcription activity was observed in crtA and it was able to be expected that the above is a cause for accumulation of SO under the condition where no As₂O₃ was added or, in other words, the background. On the other hand, even when no arsenic was added, an apparent transcription amount was able to be confirmed in arsR and, under the state where arsenic was added, transcription induction was able to be observed. Thus, mRNA of arsR which was necessary for the translation of ArsR necessary for transcription suppression in the state where no arsenic was added was detected and it was able to be expected that transcription suppression by ArsR was released by addition of arsenic and that transcription of arsR itself was also induced. That is a phenomenon which was also confirmed in *E*. *coli* [A.carkin, et al., (1995) J.Bacteriol., 177(4):981-6]. From the above, it was confirmed that transcription induction of both genes of arsR and crtA was resulted responding to the addition of arsenic and it was suggested that Pars and arsR of *E. coli* achieved the function of an arsenic-inductive promoter in *Rv. sulfidophilum* as well.

It was also evaluated that how long time was needed until the culture liquid showed red color by addition of As₂O₃. Culture liquids to which As₂O₃ was and was not added were prepared, carotenoid pigment was extracted from each of the samples after 0, 3, 6, 12, 24 and 48 hour (s) and analyzed by an HPLC and, from the area value of each peak shown in the resulting HPLC chart, rate of red pigments (DMSO and SO) to the total sum of DMSO, DMSE, SO and SE was calculated. Mean value and standard deviation for each sample were also calculated and graphs were prepared. As a result, it was found that the rate of red pigment increased from the stage after 6 hours from addition of AS₂O₃ and that the increase proceeded until 24 hours thereafter and, finally, accumulation of about 55% of red pigment was able to be confirmed. On the other hand, in case no arsenic was added, about 30% of red pigment was able to be confirmed until the stage after 6 hours from initiation of the measurement and, after that, changes were rarely noted. From those results, it was found that observation of color tone after 24 hours from addition of AS₂O₃ was the shortest time and, moreover, color tone change was most easily understood as compared with the control to which no As₂O₃ was added (Fig. 40).

### 4. Specificity of arsenic biosensor

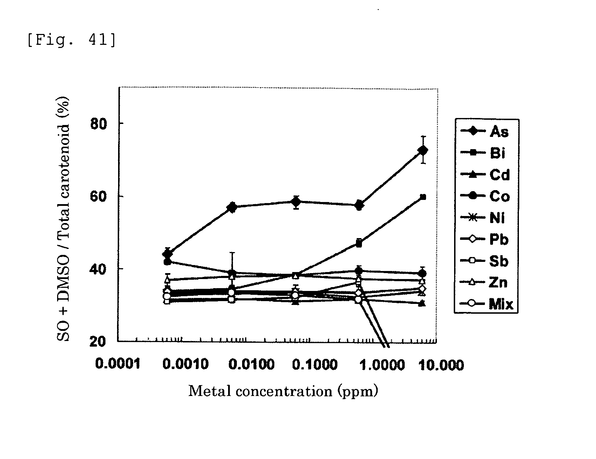

From the experiments up to now, it has been made clear that a biosensor SENS-AS strain shows a responding property to arsenic but its specificity is ambiguous. Therefore, specificity to metal of the SENS-AS strain was investigated by adding a sample containing metal other than arsenic to the SENS-AS strain and checking the responding property to those samples. With regard to the metal other than arsenic, there were used antimony and bismuth which have been reported to inherently show a responding property to ArsR and also cobalt, cadmium, nickel, zinc and lead where responding property in a protein having the similar sequence to metal-bonding site of ArsR has been confirmed already [L.S.Busenlehner, et al., (2003) FEMS Microbiol.Rev., 27:131-43]. The responding property was also tested for a solution in which iron, manganese, copper and aluminum which are abundantly contained in tap water were mixed. The experiment was carried out by the same method as in the detection of arsenic, rate of red pigments (DMSO and SO) to the total sum of DMSO, DMSE, SO and SE was calculated from the area value of each peak shown in the chart prepared as a result of an HPLC analysis, mean value and standard deviation for each sample were calculated and graphs were prepared. As a result, response was confirmed in bismuth within a concentration range of 0.6000 ppm to 6.0000 ppm but no response was found to other metals (Fig. 41).

From the above results, it was now made clear that the arsenic-detecting biosensor SENS-AS strain responded arsenic and bismuth. However, in order to respond to bismuth, a bismuth concentration of as high as 100-fold as compared with arsenic was necessary. Accordingly, the SENS-AS strain had an apparent higher specificity to arsenic than to bismuth and was found to be effective for the detection of arsenic.

### 5. Summary

Result of this Example will be as follows.
. *E. coli* Pars and arsR showed a responding ability to arsenic in *Rv. sulfidophilum* and was clarified to function as an arsenic-inductive promoter.
. By the use of *E*. *coli* Pars and arsR, and crt A of *Rv. sulfidophilum,* construction of an arsenic biosensor SENS-AS strain was in success.
. It was made clear that the SENS-AS strain responded to arsenic within a concentration range of 0.0006 ppm to 6.0000 ppm. Further, to arsenic within a concentration range of 0.0060 ppm to 0.6000 ppm, visual detection of arsenic by color tone change of the biosensor strain from yellow color to red color was possible.
. The SENS-AS strain showed a responding property to bismuth besides arsenic but, with regard to the threshold concentration for the detection, it was found that arsenic was the lowest and that specificity to arsenic was the highest.

As mentioned above, construction of an arsenic-detecting biosensor showing a responding property of arsenic was in success in this Example. Such a biosensor has a high detecting sensitivity for arsenic and the specificity is also high and, therefore, it has a property of being fully able to be utilized in a screening of contamination of arsenic in drinking water and is very useful.

Reference Documents (additional reference documents are mentioned in the text):
[1] Hidehito Yamashiro, A Graduation Thesis in 2001
[2] Y.Miura, et al., (1992) Biosci.Biotech. Biochem., 56(5):751-4
[3] R.Simon, et al., (1983) Bio/Technology, 1: 37-45
[4] V.Lorenzo, et al., (1990) J.Bacteriol., 172:6568-72
[5] Hidehito Yamashiro, A Master's Thesis in 2003

### Free text of sequence listing

SEQ ID NO. 5 is a sequence of Primer crtAdown1 described in Table 4.
SEQ ID NO. 6 is a sequence of Primer crtAdown2 described in Table 4.
SEQ ID NO. 7 is a sequence of Primer crtAdown3 described in Table 4.
SEQ ID NO. 8 is a sequence of Primer crtAup1 described in Table 4.
SEQ ID NO. 9 is a sequence of Primer crtAup2 described in Table 4.
SEQ ID NO. 10 is a sequence of Primer crtAup3 described in Table 4.
SEQ ID NO. 11 is a sequence of Primer kmR1 described in Table 4.
SEQ ID NO. 12 is a sequence of Primer kmR2 described in Table 4.
SEQ ID NO. 13 is a sequence of Primer crtArv1 described in Table 6.
SEQ ID NO. 14 is a sequence of Primer crtArv2 described in Table 6.
SEQ ID NO. 15 is a sequence of Primer crtArv3 described in Table 6.
SEQ ID NO. 16 is a sequence of Primer term1 described in Table 6.
SEQ ID NO. 17 is a sequence of Primer term2 described in Table 6.
SEQ ID NO. 18 is a sequence of Primer ddhA1 described in Table 8.
SEQ ID NO. 19 is a sequence of Primer ddhA2 described in Table 8.
SEQ ID NO. 20 is a sequence of Primer rrna1 described in Table 8.
SEQ ID NO. 21 is a sequence of Primer rrna2 described in Table 8.
SEQ ID NO. 22 is a sequence of Primer crtAup1 described in Table 8.
SEQ ID NO. 23 is a sequence of Primer crtArv3 described in Table 8.
SEQ ID NO. 24 is a sequence of Primer i-ddhA1 described in Table 8.
SEQ ID NO. 25 is a sequence of Primer i-ddhA2 described in Table 8.
SEQ ID NO. 26 is a sequence of Primer crtArv3 described in Table 10.
SEQ ID NO. 27 is a sequence of Primer pddh1 described in Table 10.
SEQ ID NO. 28 is a sequence of Primer pddh2 described in Table 10.
SEQ ID NO. 31 is a sequence of Primer E. coli-arsR-S1 described in Table 14.
SEQ ID NO. 32 is a sequence of Primer E. coli-arsR-A1 described in Table 14.
SEQ ID NO. 33 is a sequence of Primer E. coli-Pars-S1 described in Table 14.
SEQ ID NO. 34 is a sequence of Primer E. coli-Pars-A1 described in Table 14.
SEQ ID NO. 35 is a sequence of Primer crtArv4 described in Table 14.
SEQ ID NO. 36 is a sequence of Primer E.coli-arsR-A2 described in Table 16.
SEQ ID NO. 37 is a sequence of Primer crtA-RT-S1 described in Table 16.
SEQ ID NO. 38 is a sequence of Primer crtA-RT-A1 described in Table 16.
SEQ ID NO. 39 is a sequence of Primer arsR-RT-S1 described in Table 16.
SEQ ID NO. 40 is a sequence of Primer arsR-RT-A1 described in Table 16.
SEQ ID NO. 41 is a sequence of Primer rRNA-RT-S1 described in Table 16.
SEQ ID NO. 42 is a sequence of Primer rRNA-RT-A1 described in Table 16.

## Claims

1. A biosensor for the detection of a specific chemical substance, **characterized in that** it comprises a recombinant purple non-sulfur bacterium wherein sensor vector is introduced into a pigment variant of a purple non-sulfur bacterium being deficient in gene coding for a spheroidene monooxygenase enzyme, and that the sensor vector is a sensor vector where inductive promoter showing a specific response to a specific chemical substance is connected in an operable manner to the upstream region of gene coding for the spheroidene monooxygenase enzyme.

2. The biosensor according to claim 1, **characterized in that** the purple non-sulfur bacterium is *Rhodovulum sulfidophilum.*

3. The biosensor according to claim 1 or 2, **characterized in that** the specific chemical substance is dimethyl sulfide and the inductive promoter showing a response to the specific chemical substance is dimethyl sulfide-inductive promoter.

4. The biosensor according to claim 3, **characterized in that** the dimethyl sulfide-inductive promoter is a promoter which is present in an upstream region of operon of gene coding for dimethyl sulfide dehydrogenase enzyme.

5. The biosensor according to claim 4, **characterized in that** the promoter is DNA consisting of a base sequence of SEQ ID No. 1 or DNA consisting of a base sequence which is at least 60% homologous to the base sequence of SEQ ID No. 1 and having an activity of dimethyl sulfide-inductive promoter.

6. The biosensor according to claim 1 or 2, **characterized in that** the specific chemical substance is arsenic and the inductive promoter showing a response to the specific chemical substance is DNA consisting of a base sequence of SEQ ID No. 29 or DNA consisting of a base sequence which is at least 60% homologous to the base sequence of SEQ ID No. 29 and having an activity of arsenic-inductive promoter.

7. A method for the preparation of a biosensor for detection of a specific chemical substance, **characterized in that** it comprises the steps of:
preparing a pigment variant of a purple non-sulfur bacterium being deficient in gene coding for spheroidene monooxygenase enzyme;
cloning an open reading frame of gene coding for spheroidene monooxygenase enzyme being deficient in promoter on vector;
connecting an inductive promoter showing a specific response to a specific chemical substance in an operable manner to the upstream region of gene coding for spheroidene monooxygenase enzyme in the vector to construct a sensor vector; and
introducing the sensor vector into the pigment variant of a purple non-sulfur bacterium to prepare a recombinant purple non-sulfur bacterium.

8. The method for the preparation of the biosensor according to claim 7, **characterized in that** the purple non-sulfur bacterium is *Rhodovulum sulfidophilum.*

9. The method for the preparation of the biosensor according to claim 7 or 8, **characterized in that** the specific chemical substance is dimethyl sulfide and the inductive promoter showing a response to the specific chemical substance is dimethyl sulfide-inductive promoter.

10. The method for the preparation of the biosensor according to claim 9, **characterized in that** the dimethyl sulfide-inductive promoter is a promoter which is present in an upstream region of operon of gene coding for dimethyl sulfide dehydrogenase enzyme.

11. The method for the preparation of the biosensor according to claim 10, **characterized in that** the promoter is DNA consisting of a base sequence of SEQ ID No. 1 or DNA consisting of a base sequence which is at least 60% homologous to the base sequence of SEQ ID No. 1 and having an activity of dimethyl sulfide-inductive promoter.

12. The method for the preparation of the biosensor according to claim 7 or 8, **characterized in that** the specific chemical substance is arsenic and the inductive promoter showing a response to the specific chemical substance is DNA consisting of a base sequence of SEQ ID No. 29 or DNA consisting of a base sequence which is at least 60% homologous to the base sequence of SEQ ID No. 29 and having an activity of arsenic-inductive promoter.
